# EUROPEAN PATENT SPECIFICATION

(11) **EP 3 320 100 B1**
(45) Date of publication and mention of the grant of the patent: **28.08.2024**
(21) Application number: 16823589.3
(22) Date of filing: 11.07.2016
(51) Int. Cl.: C12N 15/861, A61K 39/00, C12N 15/34, C12N 5/10, C12N 7/01

(54) **FOWL ADENOVIRUS 9 (FADV-9) VECTOR SYSTEM AND ASSOCIATED METHODS**
GEFLÜGEL-ADENOVIRUS-9-VEKTORSYSTEM UND ZUGEHÖRIGE VERFAHREN
SYSTÈME DE VECTEUR À BASE D'ADÉNOVIRUS AVIAIRE 9 (FADV-9) ET MÉTHODES ASSOCIÉES

(30) Priority: 10.07.2015 US 201562190913 P
(43) Date of publication of application: 16.05.2018
(73) Proprietor: University of Guelph, Guelph, Ontario N1G 2W1 (CA)
(72) Inventor: PEI, Yanlong, Guelph, Ontario N1G 4Y6 (CA); ACKFORD, James, Whitby, Ontario L1N 9A4 (CA); CORREDOR, Juan Carlos, Ottawa, Ontario K1G1L3 (CA); KRELL, Peter J., Guelph, Ontario N1H 6H9 (CA); NAGY, Éva, Guelph, Ontario N1H 6H9 (CA)
(74) Representative: Schnappauf, Georg
(86) International application number: PCT/CA2016/050811
(87) International publication number: WO 2017/008154

(56) References cited:
- WO-A1-2010/058236
- US-B1- 6 296 852
- US-B1- 6 296 852
- US-B1- 6 335 016
- US-B1- 6 841 158
- DAVOR OJKIC ET AL: "The Long Repeat Region Is Dispensable for Fowl Adenovirus Replication in Vitro", VIROLOGY, vol. 283, no. 2, 1 May 2001 (2001-05-01), pages 197 - 206, XP055505842, ISSN: 0042-6822, DOI: 10.1006/viro.2000.0890
- JOHNSON M A ET AL: "DELIVERY OF AVIAN CYTOKINES BY ADENOVIRUS VECTORS", DEVELOPMENTAL AND COMPARATIVE IMMUNOLOGY, PERGAMON PRESS, US, vol. 24, 1 January 2000 (2000-01-01), pages 343 - 354, XP001167099, ISSN: 0145-305X, DOI: 10.1016/S0145-305X(99)00082-8
- CORREDOR J C ET AL: "The non-essential left end region of the fowl adenovirus 9 genome is suitable for foreign gene insertion/replacement", VIRUS RESEARCH, AMSTERDAM, NL, vol. 149, no. 2, 1 May 2010 (2010-05-01), pages 167 - 174, XP026966202, ISSN: 0168-1702, [retrieved on 20100202]
- L. DENG ET AL: "Oral Inoculation of Chickens with a Candidate Fowl Adenovirus 9 Vector", CLINICAL AND VACCINE IMMUNOLOGY, vol. 20, no. 8, 1 August 2013 (2013-08-01), US, pages 1189 - 1196, XP055392819, ISSN: 1556-6811, DOI: 10.1128/CVI.00187-13
- A. FRANCOIS ET AL: "Construction of Avian Adenovirus CELO Recombinants in Cosmids", JOURNAL OF VIROLOGY., vol. 75, no. 11, 1 June 2001 (2001-06-01), US, pages 5288 - 5301, XP055523857, ISSN: 0022-538X, DOI: 10.1128/JVI.75.11.5288-5301.2001
- F. L. GOFF: "Deletion of open reading frames 9, 10 and 11 from the avian adenovirus CELO genome: effect on biodistribution and humoral responses", JOURNAL OF GENERAL VIROLOGY., vol. 86, no. 7, 1 July 2005 (2005-07-01), GB, pages 2019 - 2027, XP055505844, ISSN: 0022-1317, DOI: 10.1099/vir.0.80879-0
- WASHIETL STEFAN ET AL: "Reannotation of the CELO genome characterizes a set of previously unassigned open reading frames and points to novel modes of host interaction in avian adenoviruses", BMC BIOINFORMATICS, BIOMED CENTRAL, LONDON, GB, vol. 4, no. 1, 7 November 2003 (2003-11-07), pages 55, XP021000464, ISSN: 1471-2105, DOI: 10.1186/1471-2105-4-55
- YANLONG PEI ET AL: "Fowl adenovirus 9 ORF19, a lipase homolog, is nonessential for virus replication and is suitable for foreign gene expression", VIRUS RESEARCH, vol. 260, 5 December 2018 (2018-12-05), NL, pages 129 - 134, XP055704881, ISSN: 0168-1702, DOI: 10.1016/j.virusres.2018.12.001
- PEI YANLONG ET AL: "Generation and characterization of a fowl adenovirus 9 dual-site expression vector", JOURNAL OF BIOTECHNOLOGY, ELSEVIER, AMSTERDAM, NL, vol. 266, 18 December 2017 (2017-12-18), pages 102 - 110, XP085330073, ISSN: 0168-1656, DOI: 10.1016/J.JBIOTEC.2017.12.017
- CORREDOR JUAN CARLOS ET AL: "Sequence comparison of the right end of fowl adenovirus genomes", VIRUS GENES, KLUWER ACADEMIC PUBLISHERS, BOSTON, US, vol. 36, no. 2, 17 January 2008 (2008-01-17), pages 331 - 344, XP036770439, ISSN: 0920-8569, [retrieved on 20080117], DOI: 10.1007/S11262-007-0194-9
- A. FRANCOIS ET AL: "Construction of Avian Adenovirus CELO Recombinants in Cosmids", JOURNAL OF VIROLOGY, vol. 75, no. 11, 1 June 2001 (2001-06-01), US, pages 5288 - 5301, XP055523857, ISSN: 0022-538X, DOI: 10.1128/JVI.75.11.5288-5301.2001
- CORREDOR, J. C. ET AL.: "The non-essential left end region of the fowl adenovirus 9 genome is suitable for foreign gene insertion/replacement.", VIRUS RESEARCH, vol. 149, no. 2, 2010, pages 167 - 174, XP 026966202, ISSN: 0168-1702
- CORREDOR, J.C. ET AL.: "Antibody response and virus shedding of chickens inoculated with left end deleted fowl adenovirus 9-based recombinant viruses.", AVIAN DIS., vol. 55, no. 3, 2011, pages 443 - 446, XP 055392816, DOI: 10.1637/9710-031311-Reg.1
- YANG, D.H. ET AL.: "Development of fowl adenovirus 9 based vector vaccine expressing the hemagglutinin gene of an H5N1 influenza virus", PROGRAMME AND PROCEEDINGS, P. 48. ORAL PRESENTATIONS - VACCINES AND VIRAL IMMUNOLOGY. ESW - 8 TH INTERNATIONAL CONGRESS OF VETERINARY VIROLOGY, August 2009 (2009-08-01), Budapest - Hungary, XP 008185177
- OJKIC, D ET AL.: "The Long Repeat Region Is Dispensable for Fowl Adenovirus Replication in Vitro", VIROLOGY, vol. 283, 2001, pages 197 - 206, XP 055505842, ISSN: 0042-6822
- LE GOFF, F.L. ET AL.: "Deletion of open reading frames 9, 10 and 11 from the avian adenovirus CELO genome: effect on biodistribution and humoral responses", JOURNAL OF GENERAL VIROLOGY, vol. 86, 2005, pages 2019 - 2027, XP 055505844, ISSN: 0022-1317, DOI: 10.1099/vir.0.80879-0
- DENG, L ET AL.: "Oral inoculation of chickens with a candidate fowl adenovirus 9 vector", CLIN. VACCINE IMMUNOL., vol. 20, no. 8, 2013, pages 1189 - 1196, XP 055392819, DOI: 10.1128/CVI.00187-13
- CORREDOR, J.C. ET AL.: "A region at the left end of the fowl adenovirus 9 genome that is non-essential in vitro has consequences in vivo", JOURNAL OF GENERAL VIROLOGY, vol. 91, 2010, pages 51 - 58, XP 055505850, ISSN: 0022-1317, DOI: 10.1099/vir.0.013839-0

## Description

### RELATED APPLICATION

This application claims the benefit of priority to US Provisional Patent Application No. 62/190,913 filed July 10, 2015.

### FIELD OF THE INVENTION

The present invention relates to the fowl adenovirus 9 (FAdV-9) and more particularly to a FAdV-9 dual delivery vector system and associated methods as well as use of the same for the prevention of disease.

### BACKGROUND OF THE INVENTION

Fowl adenoviruses (FAdVs) are ubiquitous poultry pathogens and are members of the family *Adenoviridae.*

Adenoviruses (AdVs) of the genus *Mastadenovirus* have been examined as anti-cancer agents (Huebner et al. (1956), Cody & Douglas (2009), Yamamoto & Curiel (2010)) and vaccine vectors (Lasaro & Ertl (2009)).

The problem of preexisting immunity against HAdV-5, exemplified in the STEP HIV trial that employed recombinant HAdV-5 (Buchbinder et al. (2008), McElrath et al. (2008)), has generated interest in the development of less common AdV serotypes and nonhuman AdVs as both oncolytic ((Cody & Douglas (2009), Gallo et al. (2005), Shashkova et al. (2005)) and vaccine vectors (Barouch (2008), Lasaro & Ertl, (2009), Sharma et al. (2009)). Fowl adenoviruses (FAdVs) of the genus *Aviadenovirus,* including species FAdV-A to FAdV-E (Adair, B. & Fitzgerald, S. (2008), Benkö et al. (2005)), are being developed as vaccine vectors. The first generation of FAdV-based vaccine vectors have proven to be effective at eliciting an antibody response against a delivered transgene (Corredor & Nagy (2010b), Ojkic & Nagy (2003)), and in chickens have conferred protective immunity against infectious bursal disease virus (IBDV) (Francois et al. (2004), Sheppard et al. (1998)) and infectious bronchitis virus (Johnson et al. (2003)). Analysis of the complete genomes of FAdV-1, the chicken embryo lethal orphan (CELO) virus (Chiocca et al. (1996)), and FAdV-9 (Ojkic & Nagy (2000)) (species FAdV-A and FAdV-D, respectively), and the terminal genomic regions of FAdV-2, -4, -10, and -8 (Corredor et al. (2006), Corredor et al. (2008)) has shown that the FAdVs share a common genome organization.

Adenovirus-based vaccine vectors have proven to be promising tools for controlling pathogens (Bangari & Mittal (2006), Ferreira et al. (2005)). The first generation of fowl adenovirus (FAdV) based vaccine vectors have been effectively used to induce an antibody response against an inserted foreign gene (transgene) (Corredor, & Nagy (2010a), Ojkic & Nagy (2003)), and in chickens have conferred protective immunity against infectious bursal disease virus (Francois et al. (2004), Sheppard et al. (1998)) and infectious bronchitis virus (Johnson et al. (2003)). US 6,296,852 relates to a recombinant vector comprising a recombinant avian adenovirus incorporating at least one heterologous nucleotide sequence, to a method of production of recombinant vectors, to methods of preparation of vaccines based on the vectors, to administration strategies and to methods of protecting poultry from disease. US 6,841,158 relates to a recombinant CELO avian adenovirus with a deletion at the right end of the viral genome allowing the insertion of large pieces of foreign DNA.

Current state of the art adenoviral vectors, especially fowl adenoviruses, are hampered by the limit of the size in the foreign DNA insert size in the vector DNA. Consequently it is not possible to clone in particularly large DNA inserts representing important parts of immunogenic proteins into an independently replicating virus. Nor it is possible to clone in more than one gene. The value of having a vector capable of expressing dual or even multivalent antigens is that only one vaccine would be needed to protect against two or more diseases. This is not possible with the current state of the art adenoviral vectors due to lack of cargo space.

Therefore, there remains a need for novel adenoviral vectors and in particular for novel dual delivery adenoviral vectors and uses thereof.

### SUMMARY OF THE INVENTION

The inventors have developed novel adenoviral vectors based on recombinant fowl adenovirus 9 (FAdV-9). The vectors are particularly useful for the delivery and/or expression of exogenous sequences and as dual delivery adenoviral vectors. Inserted into the novel vector can be one or more exogenous nucleotide sequences. Optionally, the one or more exogenous nucleotide sequences code for one or more antigenic sites of a disease of concern.

Accordingly, in a first aspect, there is provided a recombinant fowl adenovirus 9 (FAdV-9) viral vector as defined in claims 1 to 10. In one embodiment, the recombinant FAdV-9 viral vector is a dual delivery viral vector.

The FAdV-9 viral vector may have a deletion at the left end of the genome. In one embodiment, the deletion at the left end of genome comprises a deletion of one or more of ORFO, ORF1 and ORF2. The FAdV-9 viral vector has a deletion at the right end of the genome, wherein the deletion at the right end of the genome comprises at least a deletion selected from ORF19 and ORF17. In one embodiment, the FAdV-9 viral vector has deletions at both the left end and right end of the genome. In one embodiment, the FAdV-9 viral vector further has a deletion of ORFO, ORF1, ORF2, TR2, and ORF11. In one embodiment, the FAdV-9 viral vector has a deletion of ORF1, ORF2, TR2, ORF17 and ORF11. In another embodiment, the FAdV-9 viral vector has a deletion ORF1, ORF2, and ORF 19. In another further embodiment, the FAdV9-viral vector has a deletion of ORF1, ORF2, and ORF19, TR2 or ORF11.

In one embodiment, the FAdV-9 viral vector has a deletion at the left end of the genome of about 2291 base pairs, optionally between about 1900 and 2500 base pairs. In one embodiment, the FAdV-9 viral vector has a deletion at the right end of the genome of about 3591 base pairs. In one embodiment, the FAdV-9 viral vector has a deletion at the right end of the genome of between about 3000 base pairs and 4000 base pairs.

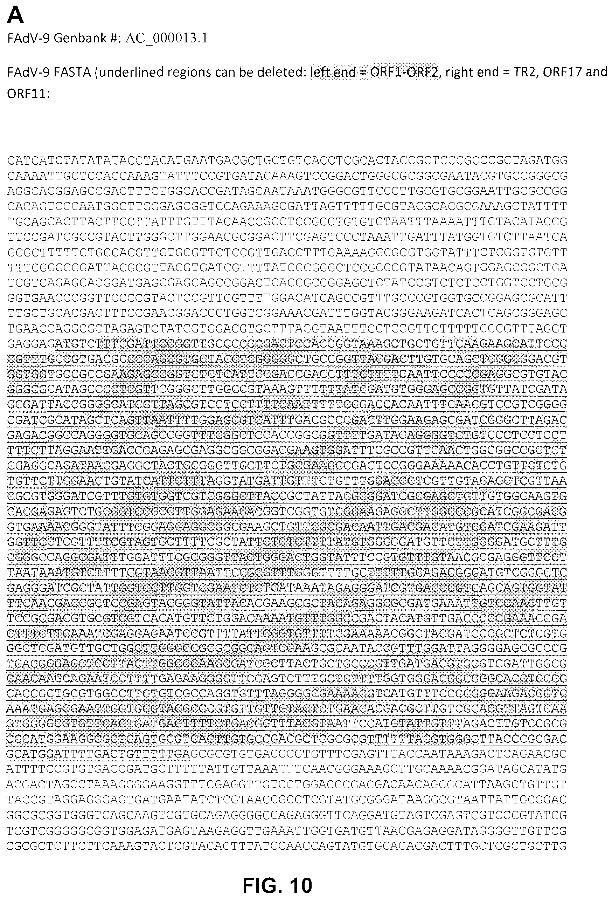

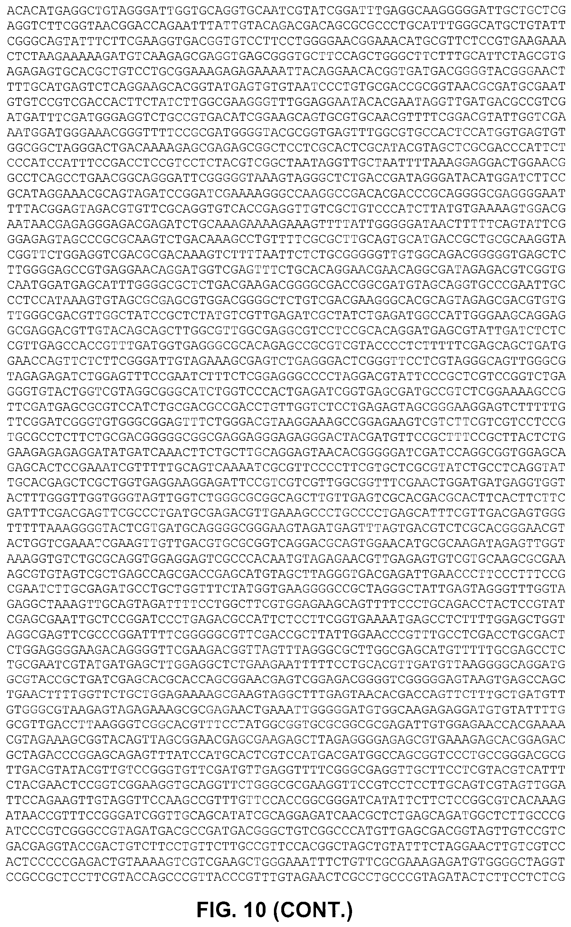

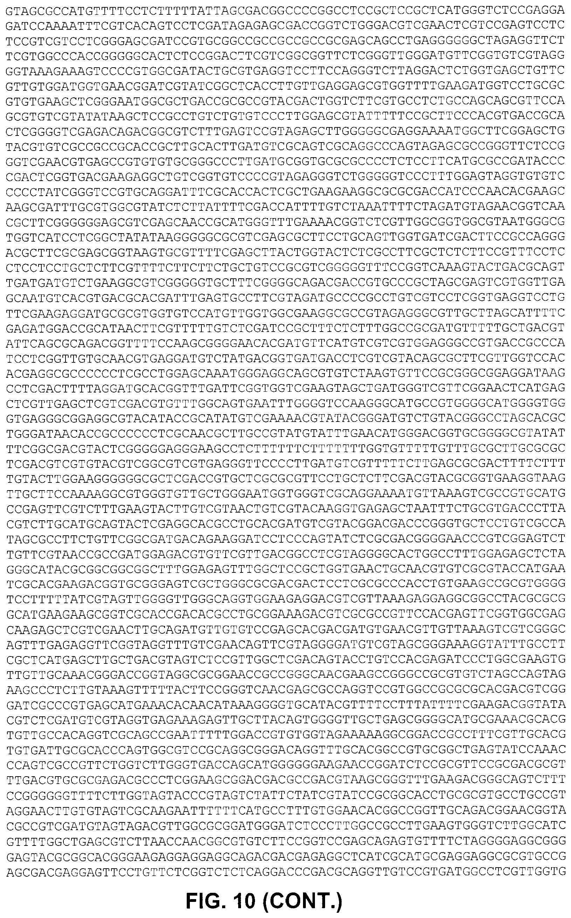

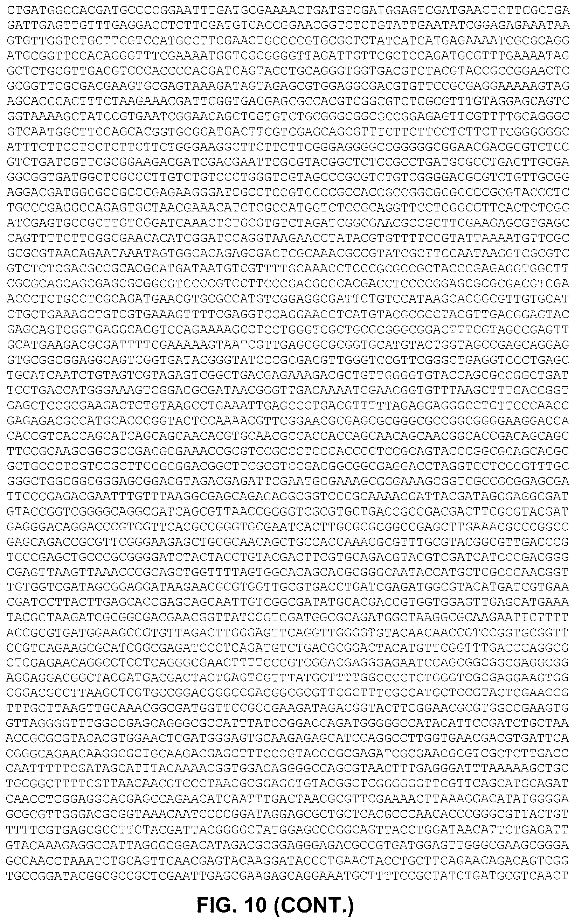

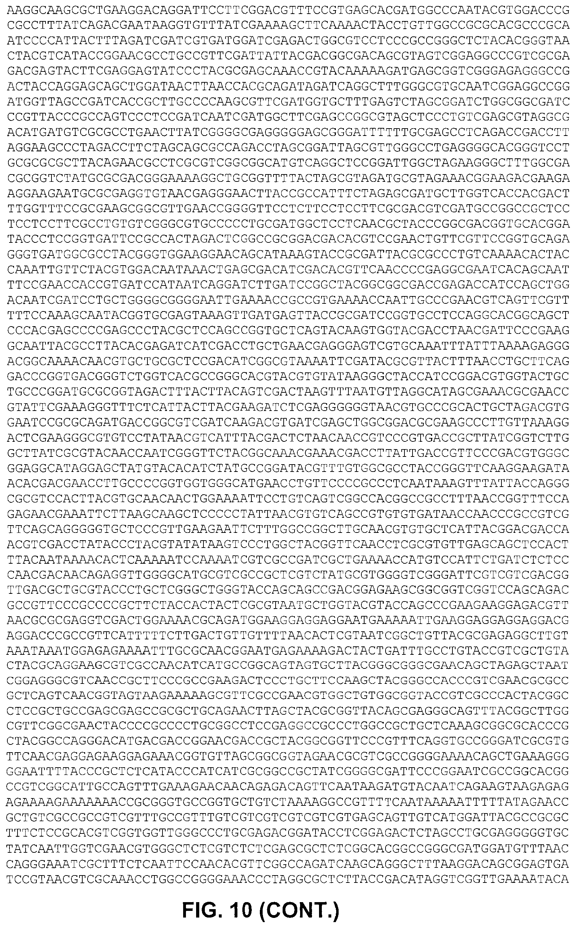

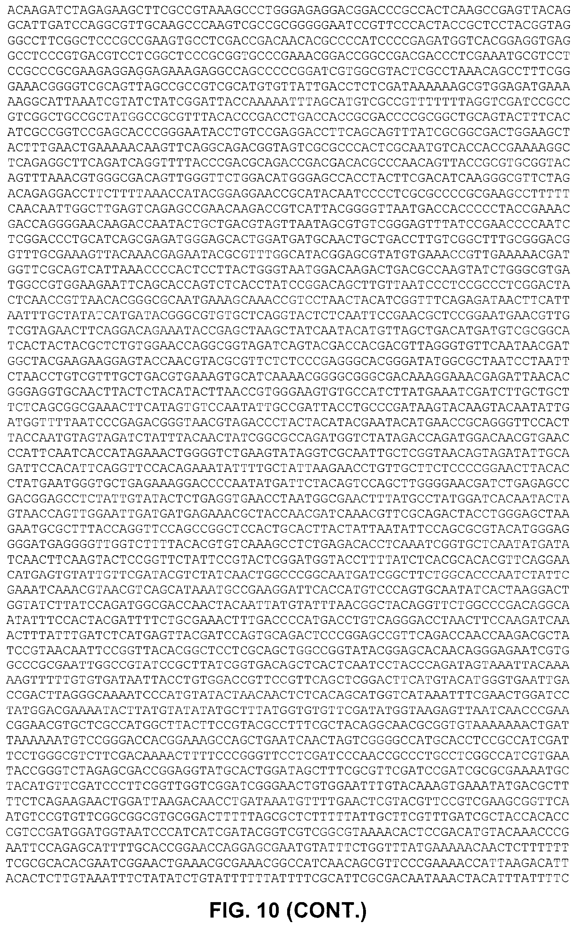

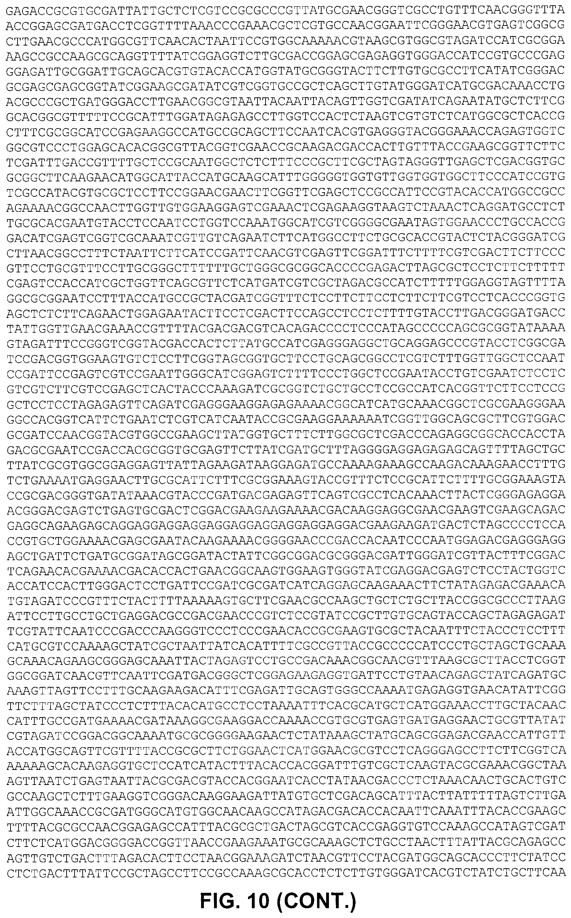

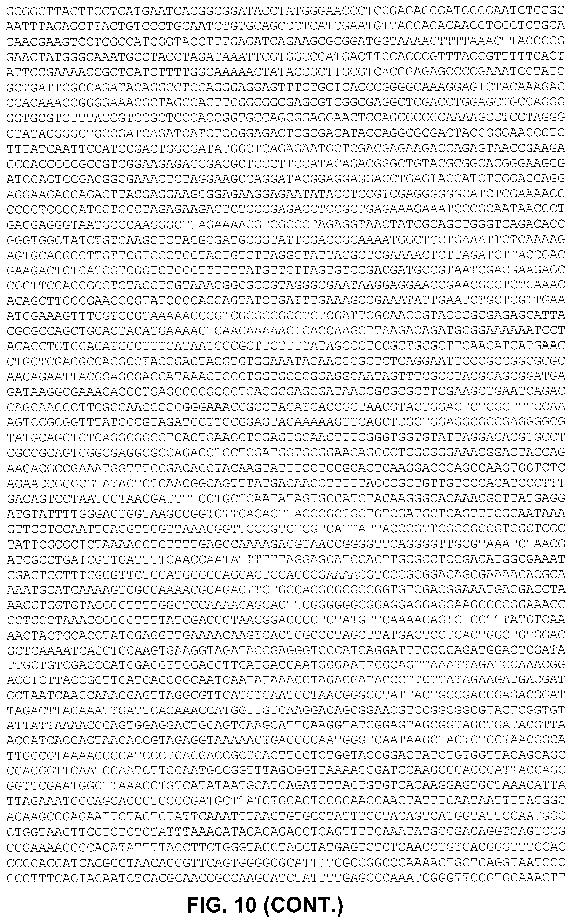

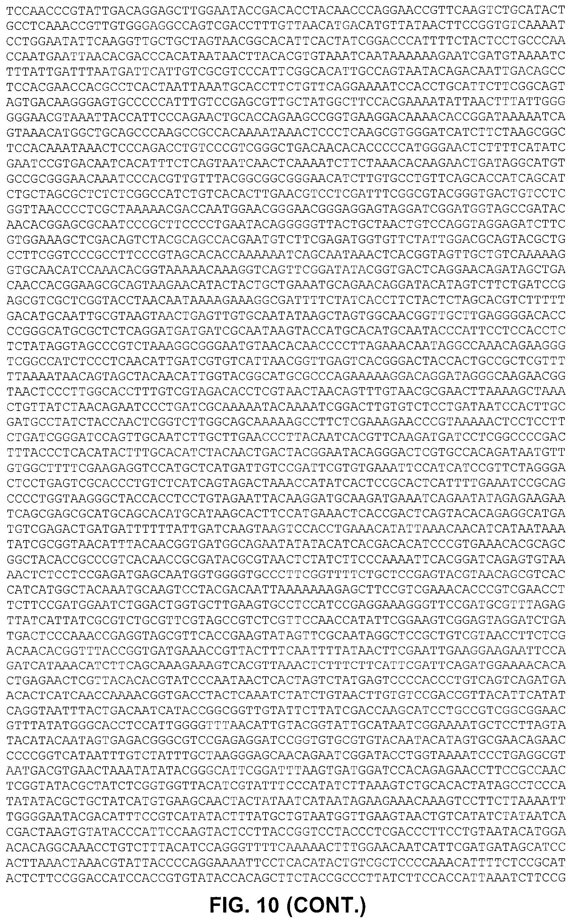

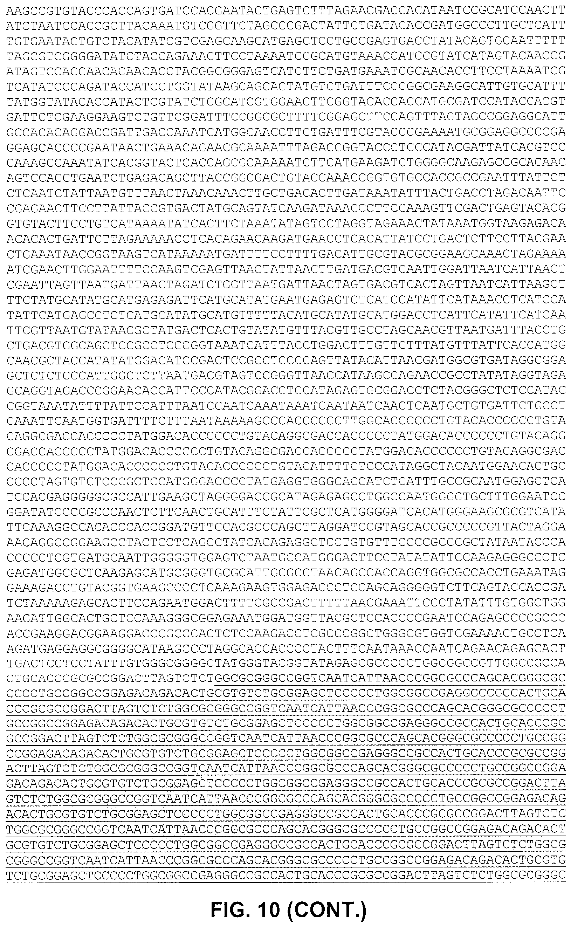

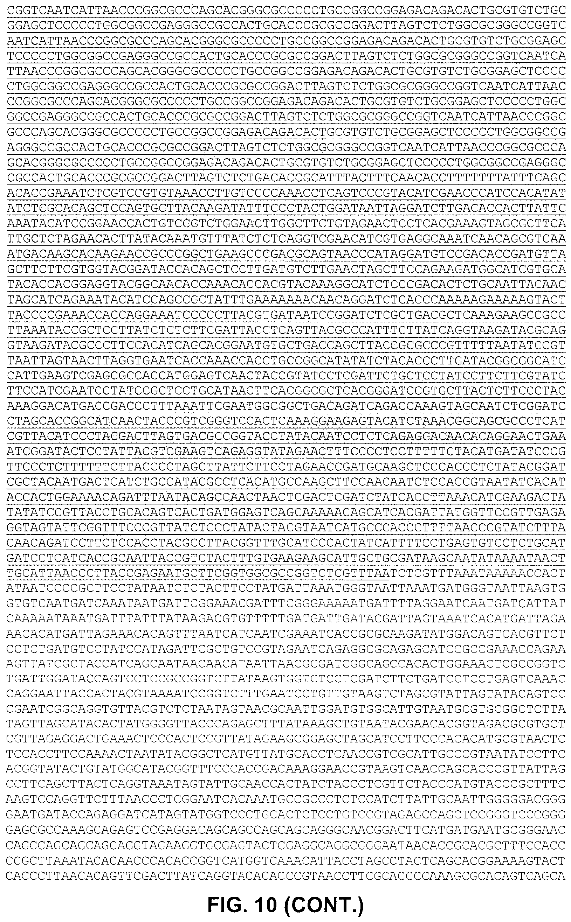

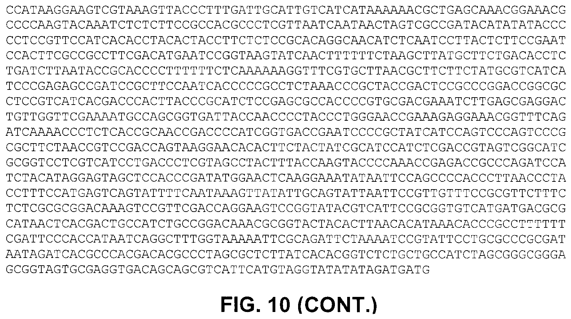

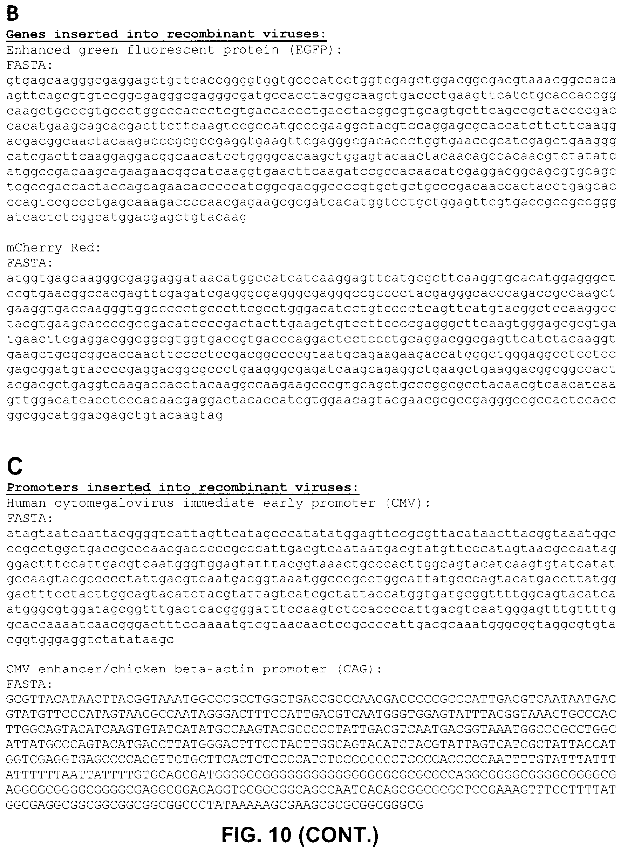

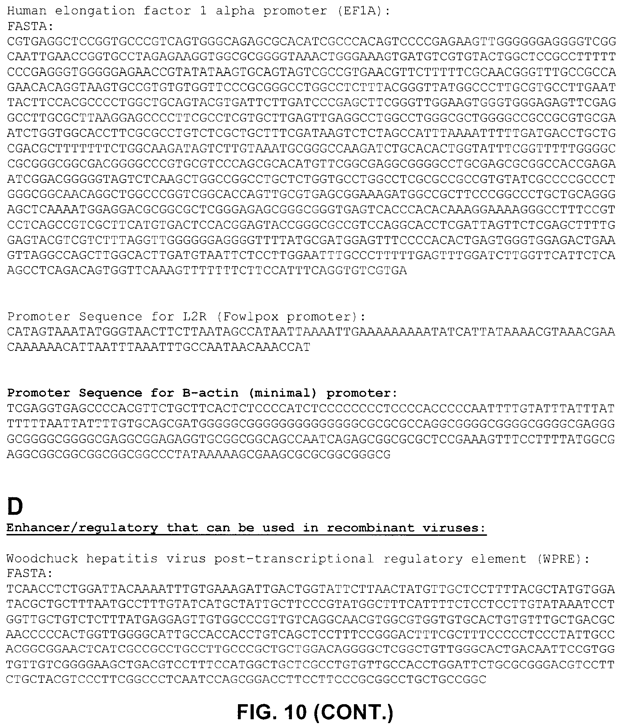

In one embodiment, the viral vector comprises a nucleotide sequence with at least 80%, at least 90%, at least 95%, at least 98% or at least 99% sequence identity to the sequence with the two deletions shown in Figure 10A. In one embodiment, the viral vector comprises or consists of the nucleotide sequence shown in SEQ ID NO: 1 with one or more deletions, optionally one or more deletions shown in Figure 10A.

In one embodiment, the viral vector has an insert capacity of greater than 4000 bp, greater than 5000 bp, greater than 6000 bp or optionally greater than 7000 bp.

In one embodiment, the viral vector comprises at least one of the following:
(a) a nucleotide sequence with sequence identity to the sequence shown in SEQ ID NO: 1, wherein nucleotides 38,807 to 42,398 have been deleted,
(b) a nucleotide sequence with sequence identity to the sequence shown in SEQ ID NO: 1, wherein nucleotides 34,220 to 36,443 have been deleted, and
(c) a nucleotide sequence with at least 70%, 75%, 80%, 85%, 90%, 95%, 96%, 97%, 98% or 99% sequence identity to the nucleotide sequences set out in (a) or (b) .

For example, in one embodiment, the viral vector comprises, consists essentially of, or consists of a nucleotide sequence with sequence identity to the sequence shown in SEQ ID NO: 1, wherein all or part of nucleotides 38,807 to 42,398 have been deleted. In one embodiment, the viral vector comprises, consists essentially of, or consists of a nucleotide sequence with sequence identity to the sequence shown in SEQ ID NO: 1, wherein all or part nucleotides 575 to 2753 and 38,807 to 42,398 have been deleted. In one embodiment, the viral vector comprises, consists essentially of, or consists of a nucleotide sequence with sequence identity to the sequence shown in SEQ ID NO: 1, wherein all or part nucleotides 847 to 2753 and 38,807 to 42,398 have been deleted. In another embodiment, the viral vector comprises, consists essentially of, or consists of a nucleotide sequence with sequence identity to the sequence shown in SEQ ID NO: 1, wherein all or part nucleotides 847 to 2753 and 34,220 to 36,443 have been deleted. In another embodiment, the viral vector comprises, consists essentially of, or consists of a nucleotide sequence with sequence identity to the sequence shown in SEQ ID NO: 1, wherein all or part nucleotides 847 to 2753, 34,220 to 36,443, and 38,807 to 40,561 or 41,461 to 42398 have been deleted.

In one embodiment, the viral vector has inserted one or more exogenous nucleotide sequences. In one embodiment, the viral vector comprises one or more exogenous nucleotide sequences coding for one or more polypeptides of interest, optionally one or more antigenic and/or therapeutic polypeptides. In one aspect, the viral vector is a dual delivery vector capable of expressing two or more exogenous nucleotide sequences. In one embodiment, the viral vector comprises exogenous nucleotide sequences coding for one or more antigenic sites of a disease of concern. In one embodiment, the viral vector comprises a sequence corresponding to at least one gene listed in Table 1 or a homolog thereof.

In a second aspect, the present invention provides host cells transformed with one or more viral vectors as described herein.

A third aspect of the invention is a method for producing a viral vector as defined in the claims, wherein the method comprises the the steps of: (a) providing the FAdV-9 viral vector of the first aspect; (b) optionally amplifying the exogenous nucleotide sequence; (c)inserting the exogenous nucleotide sequence into the viral vector; and (d) introducing the infectious clone thus produced into a suitable cell line. The exogenous nucleotide sequence may be a sequence as defined in claim 13.

In one embodiment, the FAdV-9 viral vector comprises one or more recombinant control sequences, such as one or more promoters. Optionally, the viral vector comprises one or more cloning sites to facilitate recombinant insertion of exogenous nucleotide sequences into the viral vector.

In a fourth aspect, there is provided an immunogenic composition comprising aFAdV-9 viral vector as described herein having an exogenous nucleotide sequence coding for at least one antigenic site of a disease of concern inserted therein. In one embodiment, the immunogenic composition further comprises a pharmaceutically acceptable carrier. In one embodiment, the immunogenic composition further comprises an adjuvant. In one embodiment, the immunogenic composition is a vaccine.

In a fifth aspect, there is provided an immunogenic composition of the fourth aspect for use in a method for generating an immunogenic response in a subject. In one embodiment, the method comprises administering to the subject a viral vector or immunogenic composition as described herein. In one embodiment, the methods and uses described herein are for generating an immunogenic response against a disease antigen, optionally one or more diseases listed in Table 1. In one embodiment, the methods and uses described herein are for the prevention of disease and/or vaccinating a subject against one or more diseases.

Other features and advantages of the present invention will become apparent from the following detailed description. It should be understood, however, that the detailed description and the specific examples while indicating preferred embodiments of the invention are given by way of illustration only, since various changes and modifications within the scope of the invention will become apparent to those skilled in the art from the detailed description.

### BRIEF DESCRIPTION OF THE FIGURES

The novel features of the present invention are established in the appended claims. However, the invention itself together with other objects and advantages thereof will be better understood in the following detailed description of a specific embodiment, when read along with the appended figures, in which:

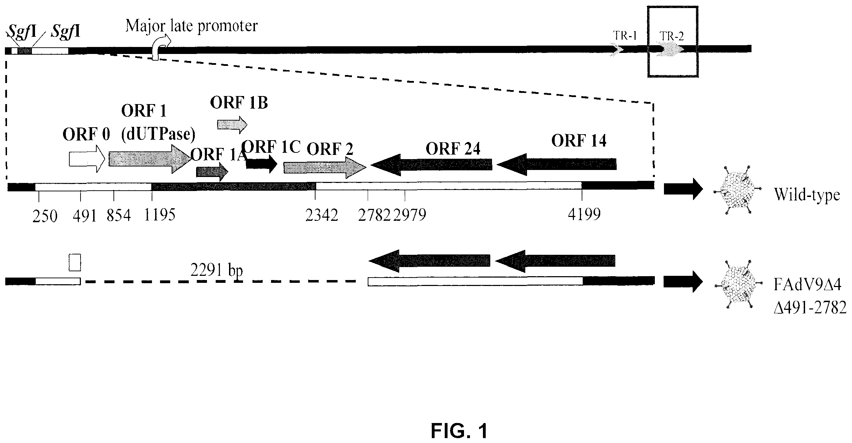

Figure 1 shows a FAdV-9 vector system (FAdmid). The Fadmid has the following characteristics: Non-pathogenic strain of FAdV-9, dispensable regions at left and right end, recombinant FAdVs grow like wild-type FAdV-9, decreased virus shedding and antibody response to viral backbone and EGFP (reporter gene) is expressed using this backbone when exogenous (foreign) promoter (CMV) is used (Corredor, J. C. & Nagy, E. (2010b)).

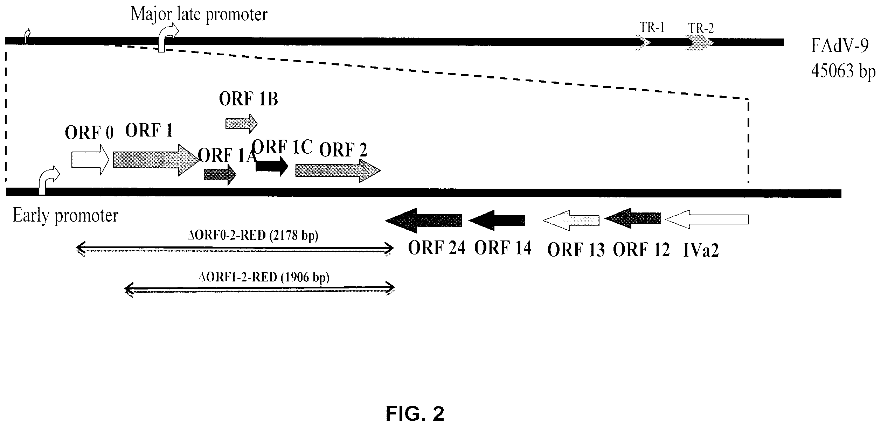

Figure 2 shows the left end sequence of FAdV-9. ORF1 and 1C function: Modulates the innate and adaptive immune response; ORFO, 1A, 1B and 2 function: Unknown. When ORF0 is deleted the native early promoter is not functioning -no foreign gene expression (e.g. m-Cherry). When only ORF 1 and 2 are deleted the native promoter works.

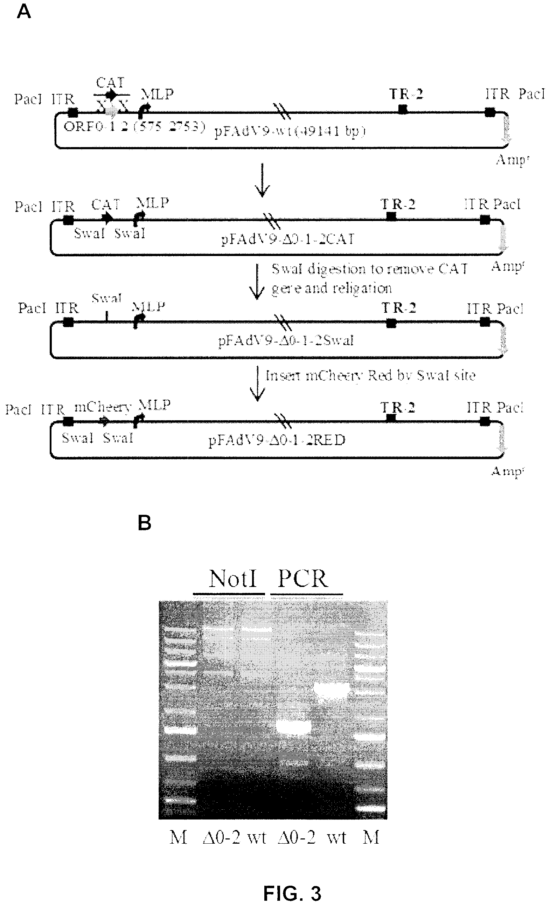

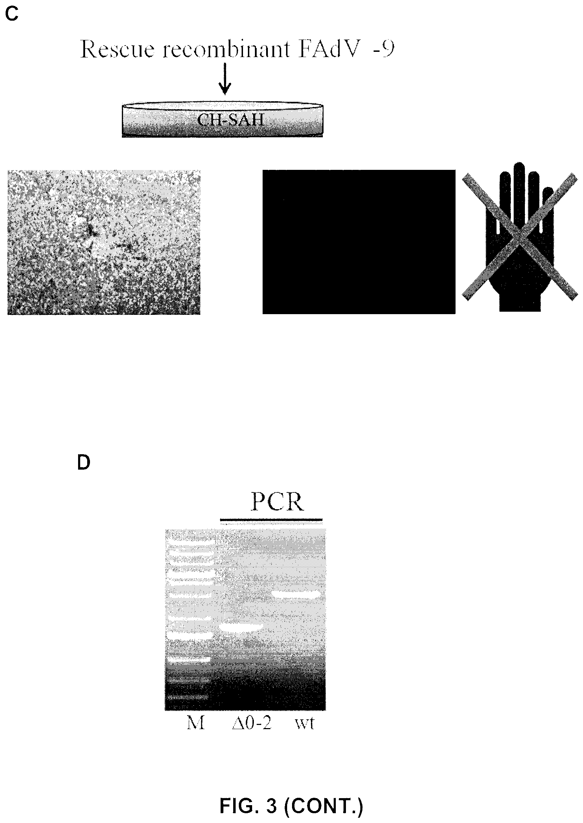

Figure 3 shows the deletion of ORFs 0, 1 and 2. (A) Flowchart of the generation of pFAdV9-Δ0-1-2-RED: ORFO, ORF1, 1A, 1B, 1C and 2 were replaced with the CAT cassette flanked on both sides with Swal sites to generate pFAdV9-Δ0-1-2CAT by homologous recombination. The CAT cassette was removed by Swal digestion followed by religation to generate unmarked pFAdV9-Δ0-1-2Swal. The mCherry coding sequence was then cloned into Swal site to generate pFAdV9-Δ0-1-2RED; MLP, major late promoter; ITR, inverted terminal repeat; (B) and (D) Notl digestions and PCR amplification of DNA to verify the FAdmids and the corresponding viruses. Lanes Δ0-2; pFAdV9-Δ0-1-2RED or passage 3 virus; lanes wt: pFAdV9-wt or virus; M: 1kb DNA ladder. Notl digestion of pFAdV9-wt DNA: fragment sizes are 26kb, 11.4 kb, 6kb, 4kb, and 1.4 kb; Notl digestion of lane Δ0-2; fragment sizes are 26kb; 11.4kb, 5.9k and 4kb; Lane Δ0-2 PCR product of 1619 pb and wt PCR product of 3107 pb; (C) Cytopathic effect (CPE) and expression of mCherry by RecFAdVs.

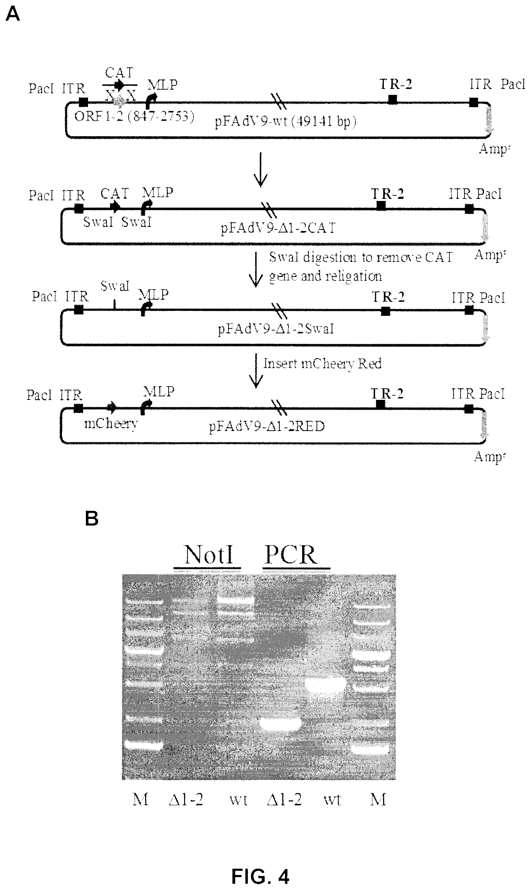

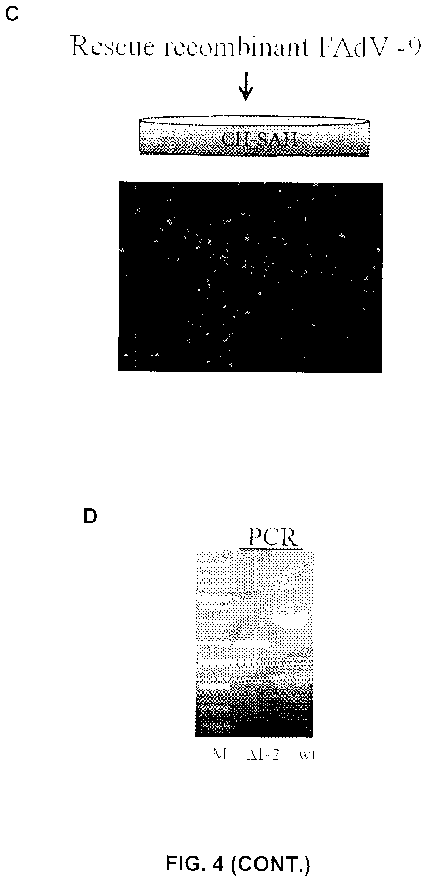

Figure 4 shows the deletion of ORFs 1 and 2. (A) Flowchart of the generation of pFAdV9-Δ1-2-RED: ORF1, 1A, 1B, 1C and 2 were replaced with the CAT cassette flanked on both sides with Swal sites to generate pFAdV9-Δ1-2CAT by homologous recombination. The CAT cassette was removed by Swal digestion followed by religation to generate unmarked pFAdV9-Δ1-2Swal. The mCherry coding sequence was then cloned into Swal site to generate pFAdV9-Δ1-2RED; MLP, major late promoter; ITR, inverted terminal repeat; (B) and (D) Notl digestions and PCR amplification of DNA to verify the FAdmids and the corresponding viruses. Lanes Δ1-2; pFAdV9-Δ1-2RED or passage 3 virus; lanes wt: pFAdV9-wt or virus; M: 1kb DNA ladder. Notl digestion of pFAdV9-wt DNA: fragment sizes are 26kb, 11.4 kb, 6kb, 4kb, and 1.4 kb; Notl digestion of lane Δ1-2; fragment sizes are 26kb; 11.4kb, 6.2k and 4kb; Lane Δ1-2 PCR product of 1912 pb and wt PCR product of 3107 pb; (C) Expression of mCherry by RecFAdVs.

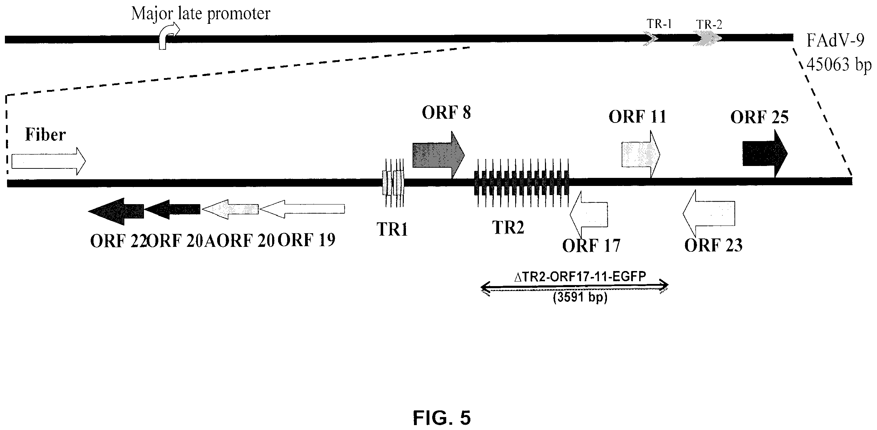

Figure 5 shows the right end sequence of FAdV-9. Function of TR-2, ORF17 and ORF11 is unknown; TR-2: the longest repeat region is composed of 13 contiguous 135-bp-long direct repeats; ORF17 and 11 are probably membrane glycoproteins.

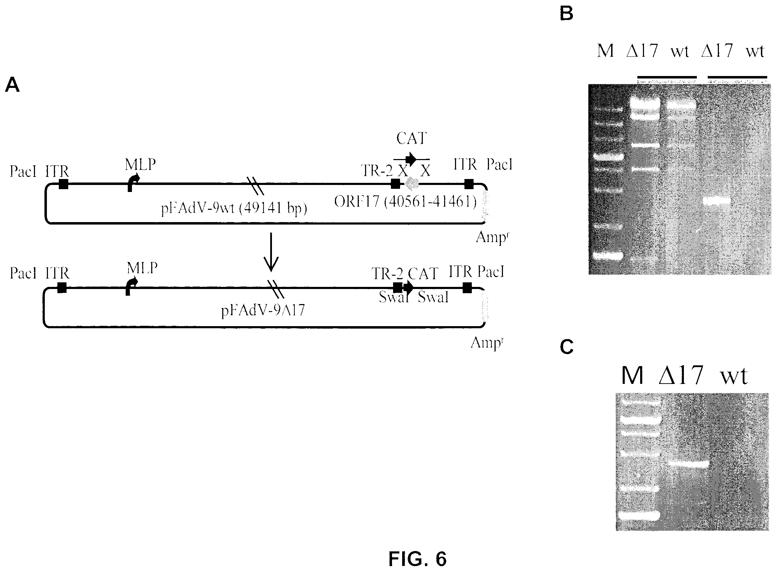

Figure 6 shows the deletion of ORF17. (A) Flow chart of the generation of pFAdV9-Δ17. ORF17 was replaced with the CAT cassette flanked on both sides with Swal sites by homologous recombination to generate pFAdV9-Δ17 (B) and (C) Notl digestions and PCR amplification of DNA to verify the FAdmids and the corresponding viruses. Lanes Δ17; pFAdV9-Δ17 or passage 3 virus; lanes wt: pFAdV9-wt or virus; M: 1kb DNA ladder. Notl digestion of pFAdV9-wt DNA: fragment sizes are 26kb, 11.4 kb, 6kb, 4kb, and 1.4 kb; Notl digestion of lane Δ17; fragment sizes are 26,381 pb; 11,485 pb, 6056 pb, 4078 pb and 1391 pb; Lane Δ17 PCR product of 2822 pb and wt PCR no product.

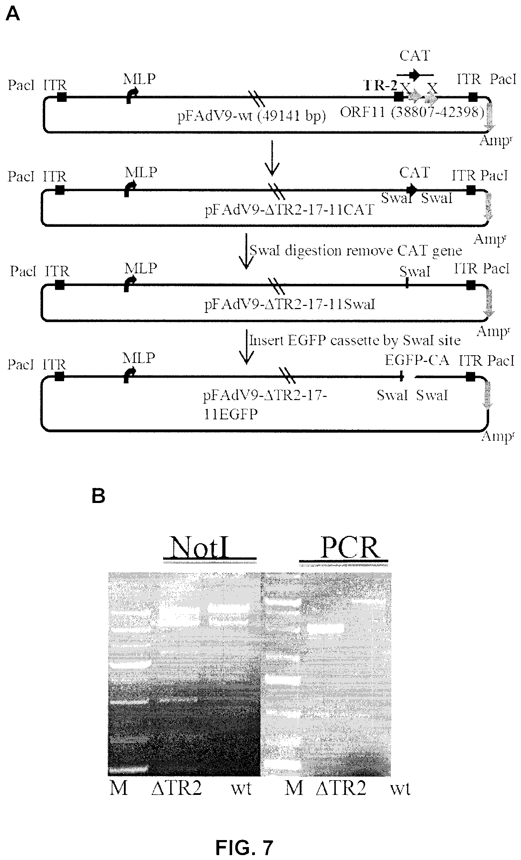

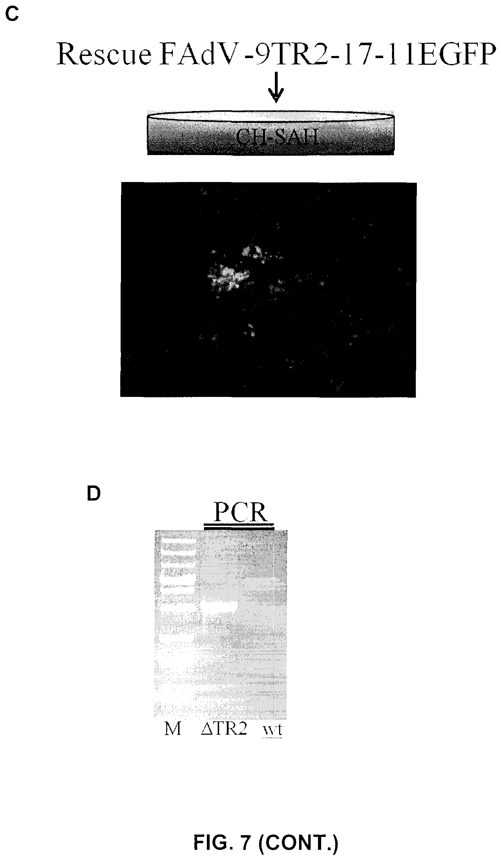

Figure 7 shows the deletion of TR2, ORFs 17 and 11. (A) Flowchart of the generation of pFAdV9-ΔTR2-17-11EGFP: TR2, ORF17, ORF11 were replaced with the CAT cassette flanked on both sides with Swal sites to generate pFAdV9-ΔTR2-17-11CAT by homologous recombination. The CAT cassette was removed by Swal digestion followed by religation to generate unmarked pFAdV9-ΔTR2-17-11Swal. The EGFP cassette was then cloned into the Swal site to generate pFAdV9-ΔTR2-17-11EGFP; MLP, major late promoter; ITR, inverted terminal repeat; EGFP cassette, CMV-EGFP; (B) and (D) Notl digestions and PCR amplification of DNA to verify the FAdmids and the corresponding viruses. Lanes ΔTR2; pFAdV9-ΔTR2-17-11EGFP or passage 3 virus; lanes wt: pFAdV9-wt or virus; M: 1kb DNA ladder. Notl digestion of pFAdV9-wt DNA: fragment sizes are 26kb, 11.4 kb, 6kb, 4kb, and 1.4 kb; Notl digestion of lane ΔTR2; fragment sizes are 21kb; 11.4kb, 6kb, 4kb, 3kb and 1.4kb; Lane ΔTR2 PCR product of 3014 pb and wt PCR product of 4966 pb; (C) Expression of mCherry by RecFAdVs.

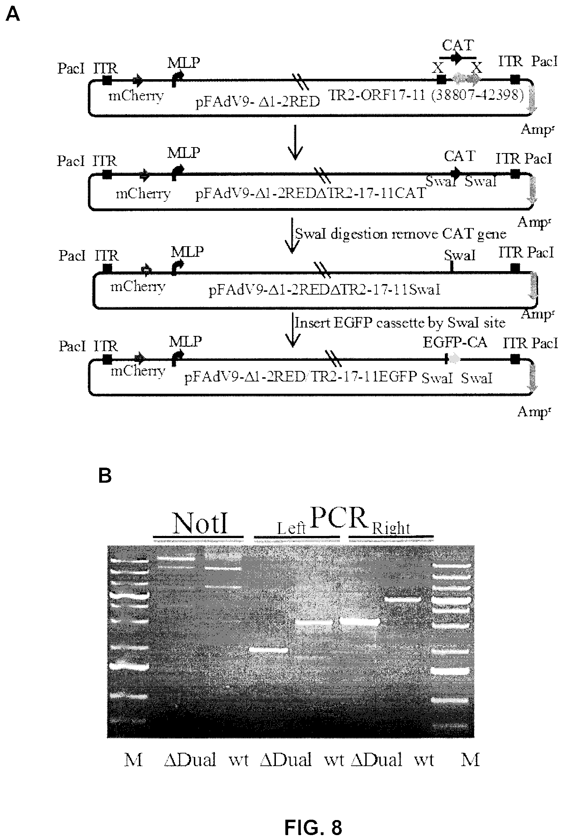

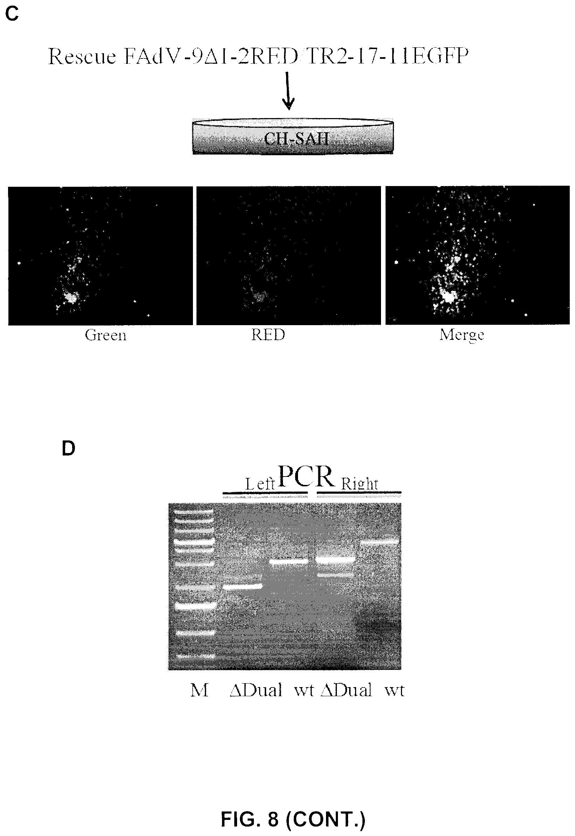

Figure 8 shows that the recombinant FAdV-9 viral vector is a dual delivery vector capable of expressing exogenous nucleotide sequences at the left end and right end. (A) Flowchart of the generation of pFAdV9-Δ1-2RED/TR2-17-11EGFP. TR2, ORF17 and ORF11 of pFAdV9-Δ1-2RED were replaced with CAT cassette flanked on both sides with Swal sites to generate pFAdV9-Δ1-2REDΔTR2-17-11CAT by homologous recombination. The CAT cassette was replaced by EGFP cassette by Swal digestion followed by ligation to generate pFAdV9-Δ1-2RED/TR2-17-11EGFP. MLP, major late promoter; ITR, inverted terminal repeat; EGFP cassette, CMV-EGFP; (B) and (D) Notl digestions and PCR amplification of DNA to verify the FAdmids and the corresponding viruses. Lanes ΔDual: pFAdV9-Δ1-2RED/TR2-17-11EGFP or passage 3 viruses. Lane M: 1kb DNA ladder. Lanes wt: pFAdV9-wt or virus. Notl digestion of pFAdV9-wt DNA: the fragment sizes are 26kb; 11.4kb, 6kb, 4kb and 1.4Kb; Lanes ΔDual Notl digestion: the fragments sizes are 21kb, 11.4kb, 6.2kb, 4kb and 3kb; Lane ΔDual left PCR product of 1912 pb and wt PCR product of 3017 pb; Lane ΔDual right PCR product of 3014 pb and wt PCR product of 4966 pb.

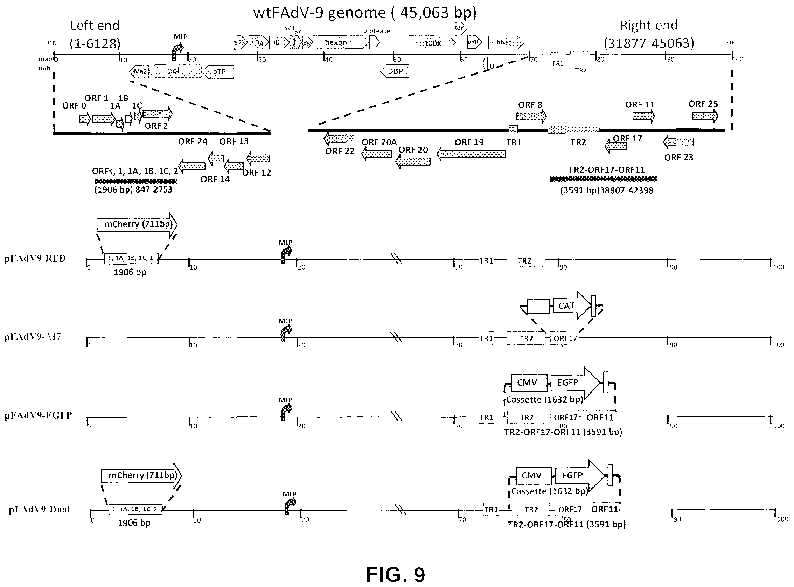

Figure 9 shows FAdV-9 based rec viruses with reporter genes.
Figure 10(A) shows the nucleotide sequence of one embodiment of a recombinant FAdV-9 viral vector as described herein wherein, ORF1, ORF2, TR2, ORF17 and ORF11 have been deleted; (B) nucleotide sequences of genes inserted into recombinant viruses: Enhanced green fluorescent protein (EGFP, SEQ ID NO: 2) and mCherry red (SEQ ID NO: 3); (C) nucleotide sequences of promoters inserted into recombinant genes: Human cytomegalovirus immediate early promoter (CMV; SEQ ID NO: 4), CMV enhancer/chicken β-actin promoter (CAG, SEQ ID NO: 5), Human elongation factor 1 alpha promoter (EF1α, SEQ ID NO: 6), L2R promoter (SEQ ID NO: 7) and β-actin promoter (SEQ ID NO: 8); and (D) nucleotide sequence of enhancer/regulatory used in recombinant viruses: Woodchuck hepatitis virus post-transcriptional regulatory element (WPER, SEQ ID NO: 9).

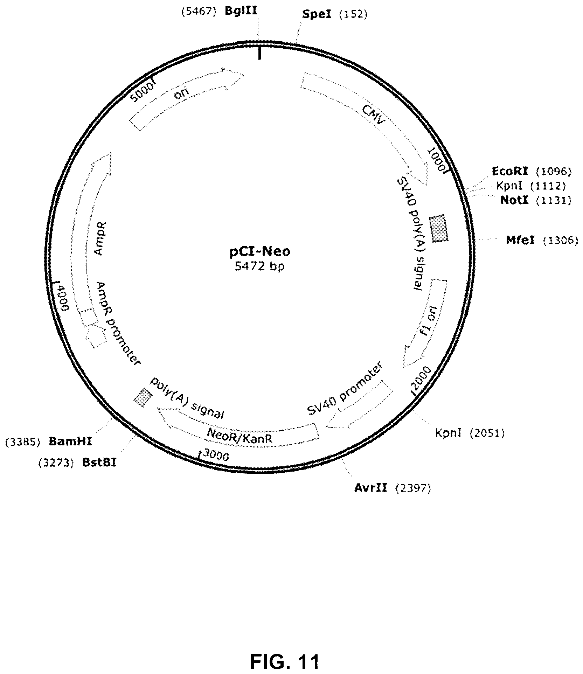

Figure 11 shows a schematic representation of pCl-Neo. The plasmid pCl-Neo (Promega) was chosen to create dual expression constructs due to its' unique restriction enzyme sites in and before the multiple cloning site, as well as the neomycin resistance gene.

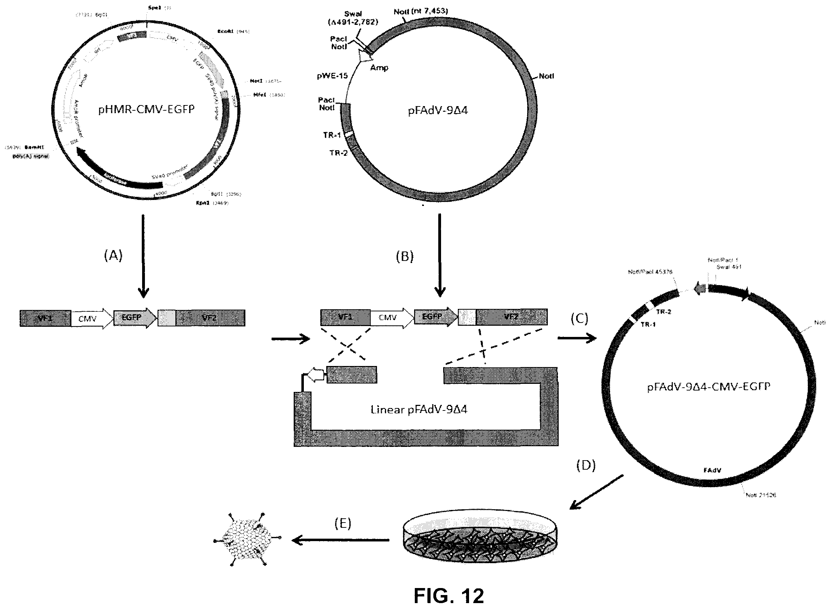

Figure 12 shows generation of recombinant FAdV-9Δ4 viruses. (A) The EGFP expression cassette was amplified by PCR from an intermediate pHMR construct, or gel extracted after double digestion with BamHI and Bglll. (B) pFAdV-9Δ4 was linearized and digested with Swal. (C) Both the EGFP cassette and the linearized pFAdV-9Δ4 was co-transformed into E. coli BJ5183 cells to undergo homologous recombination. (D) The resulting plasmid was transformed into E. coli DH5α cells, propagated, screened by Notl digestion, and eventually linearized with Pacl to release the viral genome from the plasmid. The linear recombinant viral DNA was transfected into CH-SAH cells. (E) Recombinant virus was collected in the supernatent. This procedure was carried out for each EGFP expression cassette, resulting in the viruses: FAdV-9Δ4-CMV-EGFP, FAdV-9Δ4-CMV-EGFP-WPRE, FAdV-9Δ4-CAG-EGFP, FAdV-9Δ4-CAG-EGFP-WPRE, FAdV-9Δ4-EF1α-EGFP, and FAdV-9Δ4-EF1α-EGFP-WPRE.

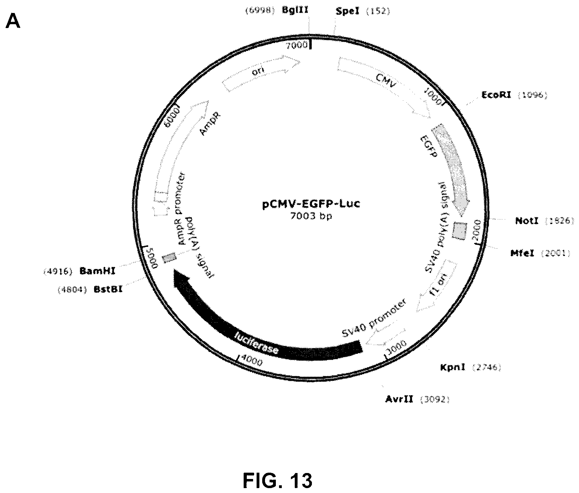

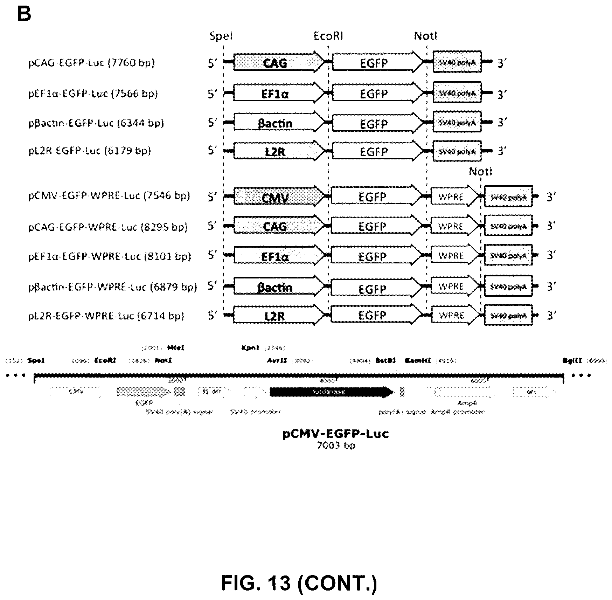

Figure 13 shows a schematic representation of EGFP/luciferase dual-expression plasmids. (A) EGFP was PCR amplified from pEGFP-N1 and cloned into the multiple cloning site of pCl-Neo. The neomycin resistance (NeoR) gene from pCl-Neo was removed by restriction enzyme digestion. Firefly luciferase was PCR amplified from pGL-4.17 and cloned under the SV40 promoter, resulting in a dual-expression plasmid. (B) The plasmid pCMV-EGFP-Luc was digested with Spel and EcoRI to clone in promoters of interest, resulting in five vectors controlling EGFP under different promoters and luciferase under the SV40 promoter: pCMV-EGFP-Luc, pCAG-EGFP-Luc, pEF1α-EGFP-Luc, pβ-actin-EGFP-Luc, and pL2R-EGFP-Luc. Five additional plasmids were made by non-directionally cloning the Woodchuck hepatitis virus post-transcriptional regulatory element (WPRE) into each dual-expression plasmid at the Notl site, resulting in the constructs: pCMV-EGFP-WPRE-Luc, pCAG-EGFP-WPRE-Luc, pEF1α-EGFP-WPRE-Luc, pβ-actin-EGFP-WPRE-Luc, and pL2R-EGFP-WPRE-Luc.

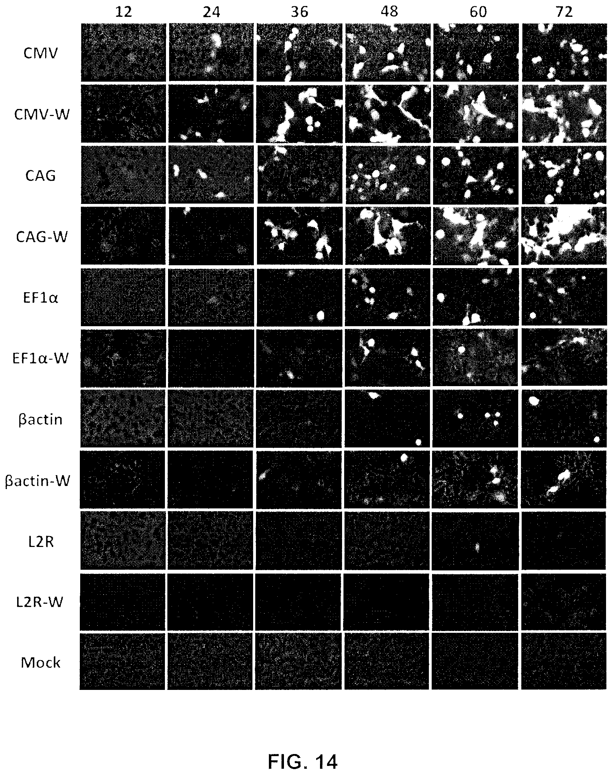

Figure 14 shows a comparison of EGFP expression with fluorescence microscopy. CH-SAH cells were seeded in 35 mm plates (1.8x10⁶ cells/dish) and transfected with 2 µg of plasmid DNA. Fluorescence of EGFP was measured by microscopy at 12, 24, 36, 48, 60, and 72 hours post-transfection. A mock transfection was used as the negative control while pEGFP-N1 was the positive control.

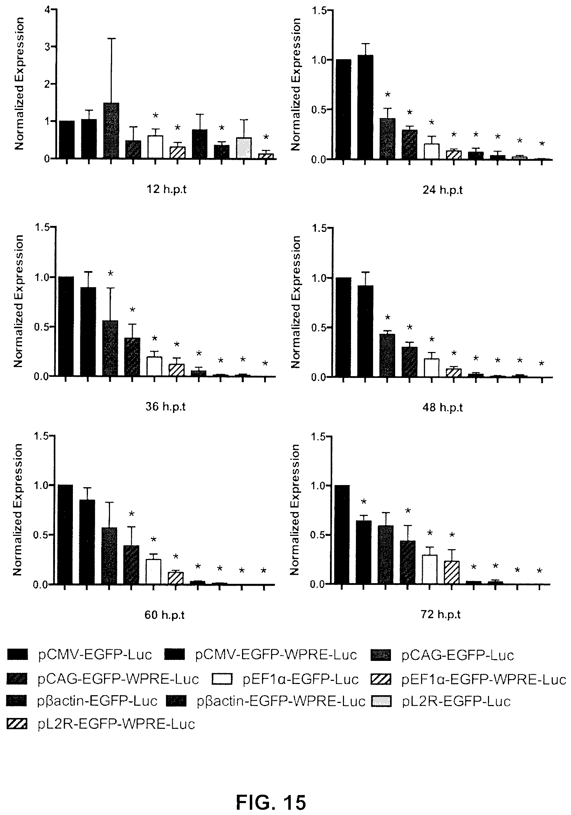

Figure 15 shows normalized EGFP expression over-time. CH-SAH cells were seeded in 35 mm plates (1.8x10⁶ cells/plate) and transfected with 2 µg of plasmid DNA. At each time-point, transfected cells were washed, trypsinized, and resuspended in PBS. After three freeze-thaw cycles, samples were centrifuged and the protein concentration of the collected supernatant was measured using a nanodrop. Fluorescence of EGFP was measured in a microplate reader at 480 and 528 nm excitation and emission wavelengths, respectively. Luminescence of luciferase was measured using a Pierce Firefly Luciferase Glow Assay kit (Thermo). The dual-expression (fluorescence/luminescence) of each construct was normalized to the expression level of pCMV-EGFP-Luc (normalized to 1.0) at each time-point. The *t*-test was used to determine the significance of expression compared to pCMV-EGFP-Luc, indicated by an asterisks (*), where P <0.05.

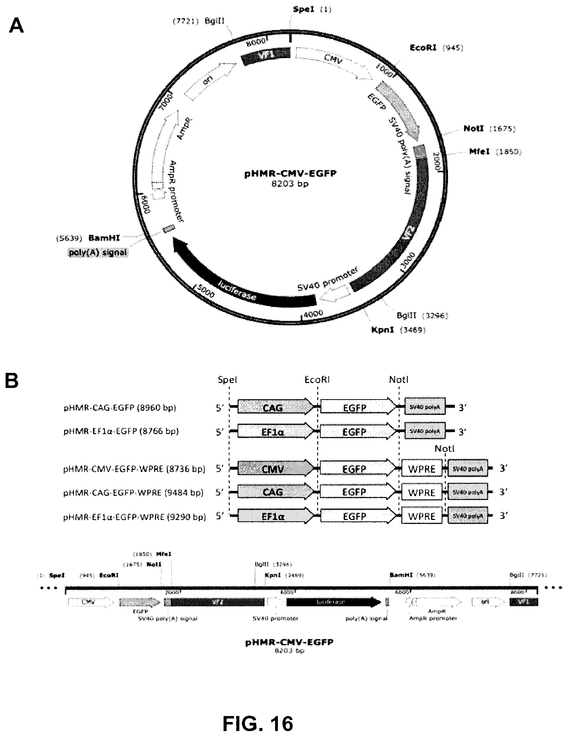

Figure 16 shows a schematic representation of intermediate constructs used to generate recFAdVs. (A) FAdV-9 DNA flanking the Δ4 deletion site (VF1 and VF2) was PCR amplified and directionally cloned into the dual-expression plasmid pCMV-EGFP-Luc. The resulting construct, pHMR-CMV-EGFP, was used downstream to generate the recombinant virus FAdV-9Δ4-CMV-EGFP. (B) Five additional intermediate constructs were generated, resulting in the plasmids: pHMR-CAG-EGFP, pHMR-EF1α-EGFP, pHMR-CMV-EGFP-WPRE, pHMR-CAG-EGFP-WPRE, and pHMR-EF1α-EGFP-WPRE.

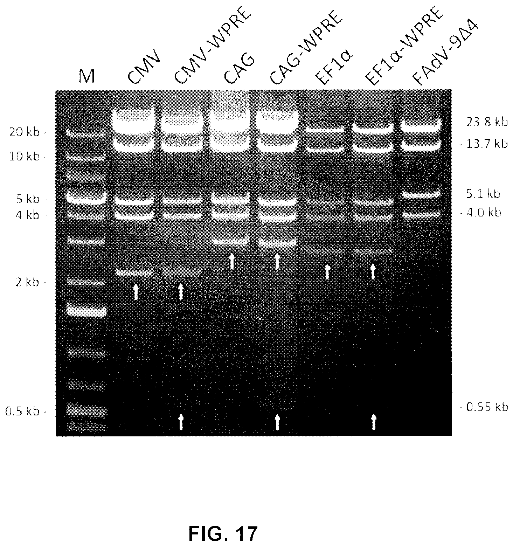

Figure 17 shows agarose gel electrophoresis screen of Notl digested FAdmids. FAdmids derived from homologous recombination between pFAdV-9Δ4 and pHMR plasmids were digested with Notl and the resulting banding patterns were screened on agarose gel. Digested pFAdV-9Δ4 has an expected banding pattern of 4 kb, 5.1 kb, 13.7 kb, and 23.8 kb. Recombinant FAdmids with an EGFP expression cassette contain an additional Notl site for screening. Diagnostic bands corresponding to each cassette are as follows: CMV-EGFP= 2.2 kb, CMV-EGFP-WPRE= 2.2 kb and 0.55 kb, CAG-EGFP= 2.9 kb, CAG-EGFP-WPRE= 0.55 kb, EF1α-EGFP= 2.7 kb, and EF1α-EGFP-WPRE= 2.7 kb and 0.55 kb. White arrows point to the diagnostic bands.

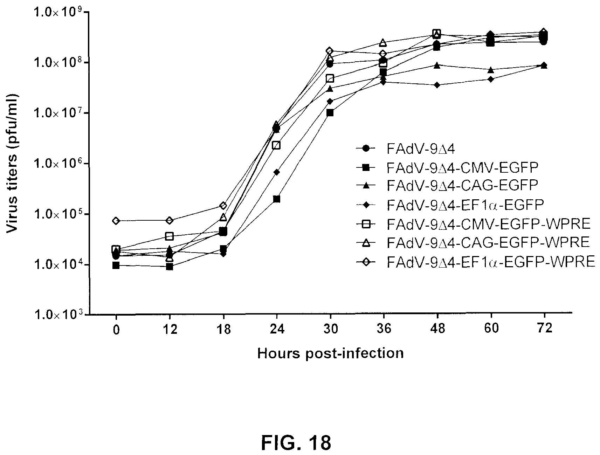

Figure 18 shows viral growth curves. One-step growth curves for each recombinant fowl adenovirus were determined in CH-SAH cells. Cells were seeded in 35 mm plates (1.8x10⁶ cells/plate) and infected at an MOI of 5. Both intracellular and extracellular virus was collected between 0-72 h.p.i. One-step growth curves were repeated in duplicate for each sample and all extracellular virus was titered by plaque assay.

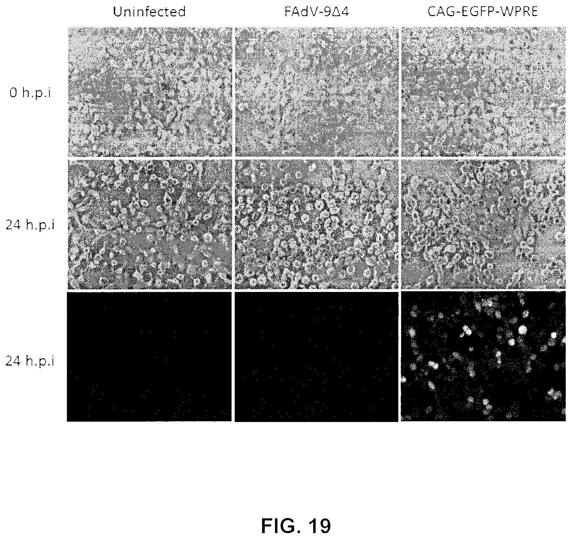

Figure 19 shows cytopathic effect of recombinant FAdV matches FAdV-9Δ4. CH-SAH cells were plated in 35 mm plates (1.8×10⁶ cells/plate) and infected at an MOI of 5. Cytopathic effect of recombinant viruses was compared to the "wild-type" control FAdV-9Δ4. It was observed that all recombinant viruses (data not shown) had CPE similar to FAdV-9Δ4, evidenced by cell rounding and detachment using a bright-field microscope. However, fluorescence of EGFP by recFAdVs, for example FAdV-9Δ4-CAG-EGFP-WPRE, was observed using fluorescence microscopy.

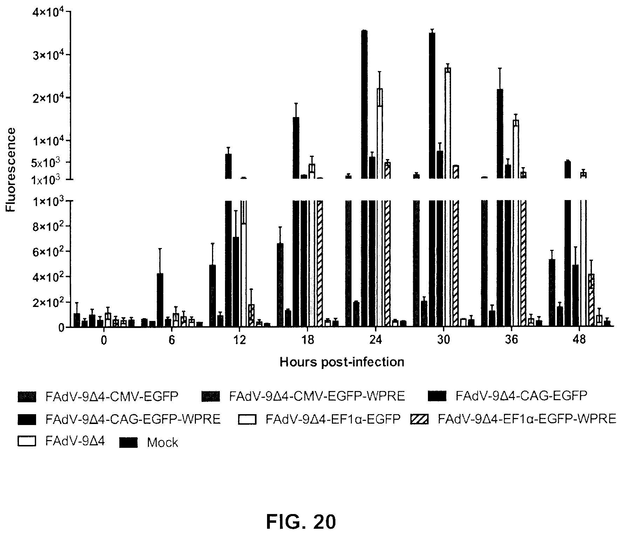

Figure 20 shows the time course of EGFP expression in CH-SAH cells. CH-SAH cells were seeded in 35 mm plates (1.8x10⁶ cells/plate) and infected with recFAdV-9s at an MOI of 5. At each time-point, infected cells were collected, washed, and resuspended in PBS. After three freeze-thaw cycles, samples were centrifuged and the protein concentration of the collected supernatant was measured with a nanodrop. The absolute fluorescence of EGFP from 50 µg of whole cell lysate was measured with a microplate reader at 480 and 528 nm excitation and emission wavelengths, respectively. Uninfected (mock) or FAdV-9Δ4 infected cells did not show any EGFP fluorescence.

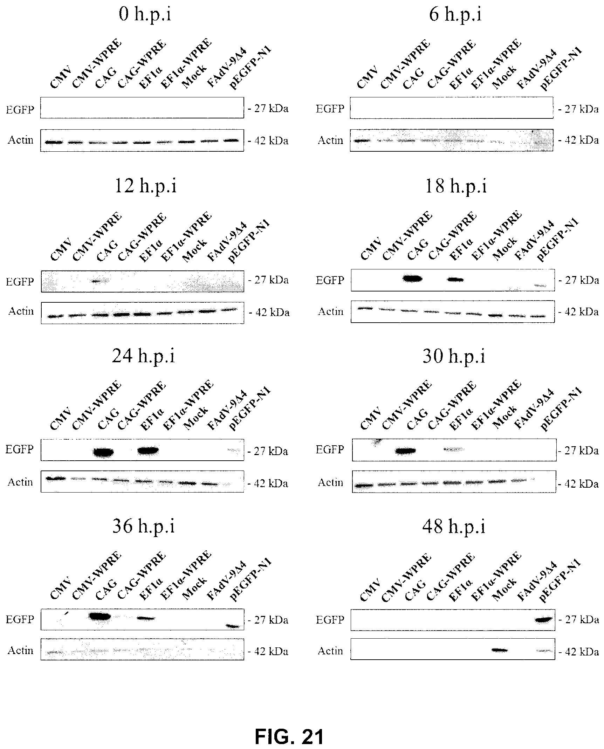

Figure 21 shows western immunoblot of EGFP production over-time. EGFP expression by recFAdV-9s in CH-SAH cells was compared over 48 hpi, along with FAdV-9Δ4 (negative control), uninfected mock (negative control), and pEGFP-N1 transfected CH-SAH cells (positive control). CH-SAH cells were seeded in 35 mm plates (1.8x10⁶ cells/dish) and infected with recFAdV-9s at an MOI of 5. At each time-point whole cell lysates were collected and protein concentrations were determined by Bradford assay. Six µg of each sample was loaded into two gels, one to be probed with anti-EGFP antibody (1:1,000) followed by a secondary anti-mouse HRP conjugated antibody (1:5,000), the other gel to be probed with anti-actin antibody (1:200) followed by a secondary anti-goat HRP conjugated antibody (1:20,000). All bands appeared at the expected size (EGFP = 27 kDa and actin = 42 kDa) as determined from a molecular weight marker (not shown).

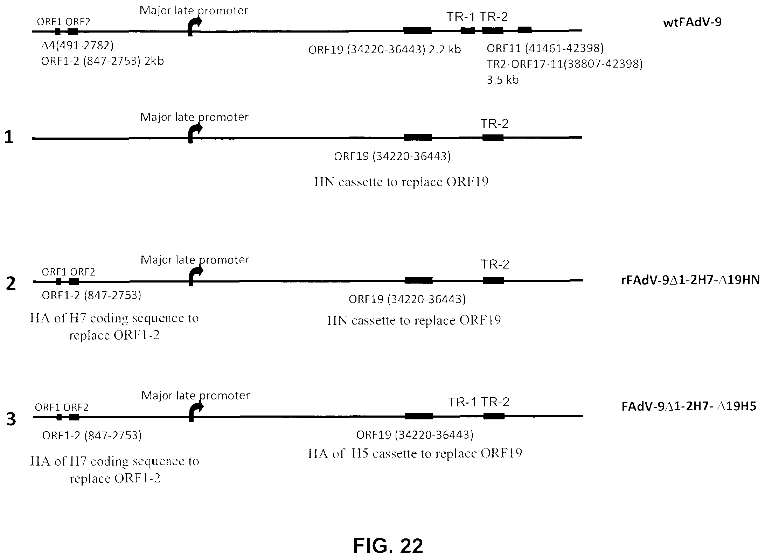

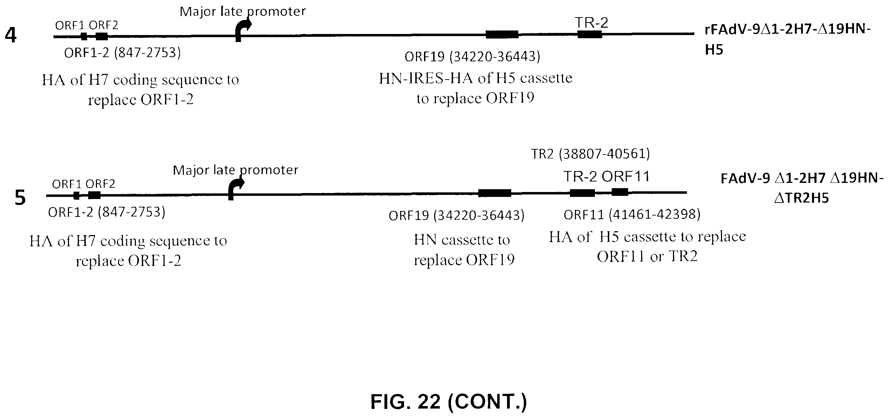

Figure 22 shows polyvalent recombinant FadV-9 with H5, H7 and HN.

### DETAILED DESCRIPTION OF THE INVENTION

The inventors have determined that recombinant FAdV-9 with a deletion on the right side of the genome, i.e., a deletion selected from ORF19 and ORF17, are useful as viral vectors.

In one embodiment of the invention, the recombinant FAdV-9 viral vector further has a deletion at the left end of the genome. In one embodiment, the deletion at the left end of genome comprises a deletion of one or more of ORFO, ORF1 and ORF2.

In one embodiment, the deletion at the right end of the genome comprises a deletion of ORF19, TR2, ORF17 and ORF11.

In one additional embodiment, the recombinant FAdV-9 viral vector has deletions at both the left end and right end of the genome. In one embodiment, the FAdV-9 viral vector has a deletion of ORFO, ORF1, ORF2, TR2, ORF17 and ORF11. In one embodiment, the recombinant FAdV-9 viral vector has a deletion of ORF1, ORF2, TR2, ORF17 and ORF11. In one embodiment, the FAdV-9 viral vector has a deletion of ORF1, ORF2, and ORF 19. In one embodiment, the recombinant FAdV9-viral vector has a deletion of ORF1, ORF2 and ORF19, TR2 or ORF11.

As shown in the Examples, recombinant FAdV-9 viral vectors with deletions on the left side and right side, including deletion of TR2, are stable and may be used to drive transgene expression.

The FAdV viral vectors described herein provide a number of advantages. For example, in some embodiments the FAdV viral vectors allow for the production of mono and polyvalent vaccines. The value of having a vector capable of expressing dual or even multivalent antigens is that only one vaccine would be needed to protect against to ore more diseases. In one embodiment, the vectors are capable of incorporating large segments of foreign DNA, optionally up to 7.7 kb of foreign DNA. In one embodiment, the vectors are stable and safe for use in animals such as chickens. In one embodiment, viral vectors are easy to produce, and produce high viral titers. In one embodiment, there are a variety of serotypes of FAdVs. In one embodiment, the viral vectors have no pre-existing immunity in humans and may be used in human gene therapy.

In one embodiment, the FAdV-9 viral vectors described herein may include one or more exogenous nucleotide sequences (also referred to herein as transgenes).

In an embodiment of the invention, the exogenous nucleotide sequence is selected from antigenic sequences against influenza, infectious laryngotracheitis, infectious bronchitis, bursa of Fabricius' infection (Gumboro), hepatitis, viral rhinotracheitis, infectious coryza, Mycoplasma hyopneumonieae, pasteurellosis, Porcine Respiratory and Reproductive Syndrome (PRRS), circovirus, bordetellosis, parainfluenza, or any other antigen which size allows its insertion into the corresponding viral vector.

In another embodiment, the exogenous nucleotide sequence is selected from antigenic sequences against Avian influenza, Laryngotracheitis (LT), Newcastle disease (NDV), infectious anemia, Inclusion bodies, Infectious Bronchitis (IB), Metapneumovirus (MPV) or Gumboro.

In one preferred embodiment of the invention, the exogenous nucleotide sequence comprises, consists essentially of or consists of a sequence corresponding to at least one gene disclosed in Table 1, or a homolog thereof. As used herein, the term "homolog" of a gene is intended to denote a gene with at least 85%, of at least 90%, at least 98% or at least 99% sequence identity to the gene, and having a biological activity of the same nature. In one embodiment, the vector described herein comprises 2, 3 or 4 sequences corresponding to the genes disclosed in Table 1, or a homolog thereof. Optionally, the exogenous sequences may be inserted into the vector at a single insertion site or multiple different insertions sites.

**Table 1.**

| **DISEASE** | **GENE** | **GEN BANK ACCESSION** |
|---|---|---|
| Avian influenza | Mexican Avian influenza H5N2 hemagglutinin, strain 435 | FJ864690.1 (SEQ ID NO: 10) |
| | Avian influenza H7N3 hemagglutinin Jalisco 2012 | JX397993.1 (SEQ ID NO: 11) |
| | Avian influenza H7N3 hemagglutinin Guanajuato 2015 | KR821169 (SEQ ID NO: 12) |
| Laryngotracheitis (LT) | Laryngotracheitis glycoprotein B, strain USDA | EU104965.1 (SEQ ID NO: 13) |
| | Laryngotracheitis glycoprotein D, strain USDA | JN542534.11:132317-133621 (SEQ ID NO: 14) |
| Newcastle (NDV) | HN gene strain LaSota | \|KC844235.11:6412-8145 (SE ID NO: 15) |
| | HN gene strain Newcastle disease virus isolate Chicken/M08-3313/Mexico/2008 | KF910962.1 (SEQ ID NO: 16) |
| infectious anemia | VP1 gene from strain Chicken anemia virus isolate CAV-10 | KJ872513.1\|:832-2181 (SEQ ID NO: 17) |
| | VP2 gene from strain Chicken anemia virus isolate CAV-10 | AIV09094.1 (SEQ ID NO: 18) |
| Inclusion bodies | Fowl adenovir us 4 short fiber gene | AY340863.1 (SEQ ID NO: 19) |
| Infectious Bronchitis (IB) | Infectious bronchitis virus strain ArkDPI-derived commercial vaccine D spike glycoprotein gene | EU359651.1 (SEQ ID NO: 20) |
| Metapneumovirus (MPV) | Avian metapneumovirus isolate IT/Ty/A/259-01/03, F gene | \|JF424833.1\|:2943-45599 (SEQ ID NO: 21) |
| Gumboro | Gumboro VP2 | |

The FadV-9 viral vectors described herein can be prepared using recombinant technologies such as PCR amplification of a nucleotide sequence of interest, by identifying the antigenic sites from an isolation of the origin-pathogen, to be further inserted, amplified in the viral vector. The insertion may be made using standard molecular biology techniques, such as restriction enzymes and DNA ligases, amongst others. The infectious clone thus produced is introduced into a suitable cell line for the production of the recombinant virus. For example, in one embodiment, the methodologies required for the construction of a FAdV-9 viral vectors are described in the present Examples and the procedures described for the construction of the FAdV-9 infectious clone (FAdmid) (Ojkic, D. & Nagy, E. (2001), The long repeat region is dispensable for fowl adenovirus replication in vitro. Virology 283, 197-206). This procedure utilizes homologous recombination between the viral genomic DNA and the linearized plasmid containing both ends of the genome flanking a backbone vector (pWE-Amp with Pacl sites introduced). Next, a foreign gene of interest with a promoter to drive its expression is inserted in suitable genomic regions of the infectious clone that are dispensable or non-essential (Corredor and Nagy, 2010a and 2010b), and introduced into a suitable cell line.

The viral vector of the present invention can be used, for example, for the preparation and administration of immunogenic compositions comprising at least the viral vector as described herein and an exogenous nucleotide sequence coding for at least one antigenic site of a disease of concern inserted therein. Optionally, the viral vector of the present invention is a dual delivery vector that can be used to drive the expression of two or more exogenous nucleotide sequences. In one embodiment, the two or more exogenous nucleotide sequences are under the control of different promoters. In one embodiment the promoters are selected from the group consisting of CMV, CAG, EF1α, β-actin and L2R.

In one embodiment, the fowl adenovirus described herein comprises a nucleotide sequence with sequence identity to the sequence shown in SEQ ID NO: 1, wherein ORFO, ORF1, ORF2, TR2, ORF17 and ORF11 have been deleted. SEQ ID NO: 1 corresponds to the complete genome sequence of Fowl adenovirus D (Genbank accession no. AC_000013.1). In one embodiment, the FAdV-9 viral vector described herein comprises or consists of a nucleotide sequence with at least 70%, 75%, 80%, 85%, 90%, 95%, 96%, 97%, 98%, 99%, or 100% sequence identity to the sequence shown in SEQ ID NO: 1, wherein all or part of ORFO, ORF1, ORF2, TR2, ORF17 and ORF11 have been deleted. In a preferred embodiment, the fowl adenovirus described herein comprises a nucleotide sequence with sequence identity to the sequence shown in SEQ ID NO: 1, wherein all of part of ORF1, ORF2, TR2, ORF17 and ORF11 have been deleted. In one embodiment, the FAdV-9 viral vector described herein comprises or consists of a nucleotide sequence with at least 70%, 75%, 80%, 85%, 90%, 95%, 96%, 97%, 98%, 99%, or 100% sequence identity to the sequence shown in SEQ ID NO: 1, wherein ORF1, ORF2, TR2, ORF17 and ORF11 have been deleted.

In another embodiment, the fowl adenovirus described herein comprises a nucleotide sequence with sequence identity to the sequence shown in SEQ ID NO: 1, wherein all or part of ORF1, ORF2 and ORF19 have been deleted. In one embodiment, the FAdV-9 viral vector described herein comprises or consists of a nucleotide sequence with at least 70%, 75%, 80%, 85%, 90%, 95%, 96%, 97%, 98%, 99%, or 100% sequence identity to the sequence shown in SEQ ID NO: 1, wherein all or part of ORF1, ORF2, and ORF19 have been deleted.

In another embodiment, the fowl adenovirus described herein comprises a nucleotide sequence with sequence identity to the sequence shown in SEQ ID NO: 1, wherein all or part of ORF1, ORF2 and ORF19, TR2 or ORF11 have been deleted. In one embodiment, the FAdV-9 viral vector described herein comprises or consists of a nucleotide sequence with at least 70%, 75%, 80%, 85%, 90%, 95%, 96%, 97%, 98%, 99%, or 100% sequence identity to the sequence shown in SEQ ID NO: 1, wherein all or part of ORF1, ORF2 and ORF19, TR2 or ORF11 have been deleted.

In another embodiment, the fowl adenovirus described herein comprises a nucleotide sequence with sequence identity to the sequence shown in SEQ ID NO: 1, wherein all or part of nucleotides 38,807 to 42,398 have been deleted. This nucleotide sequence (nucleotides 38,807 to 42,398) includes TR-2, ORF17 and ORF11. In another embodiment, the FAdV-9 viral vector described herein comprises or consists of a nucleotide sequence with at least 70%, 75%, 80%, 85%, 90%, 95%, 96%, 97%, 98%, 99%, or 100% sequence identity to the sequence shown in SEQ ID NO: 1, wherein all or part of nucleotides 38,807 to 42,398 have been deleted.

In one embodiment, the viral vector comprises or consists of a nucleotide sequence with sequence identity to a sequence comprising or consisting of SEQ ID NO: 1, wherein at least 500, 1000, 1500, 2000, 2500, 3000, 3500, 4000, 4500 or 5000 nucleotides corresponding to all or part of nucleotides 575 to 2753 and 38,807 to 42,398 of SEQ ID NO: 1 have been deleted.

In one embodiment, the viral vector comprises or consists of a nucleotide sequence with sequence identity to a sequence comprising or consisting of SEQ ID NO: 1, wherein at least 500, 1000, 1500, 2000, 2500, 3000, 3500, 4000, 4500 or 5000 nucleotides corresponding to all or part of nucleotides 847 to 2753 and 38,807 to 42,398 of SEQ ID NO: 1 have been deleted.

In another embodiment, the viral vector comprises or consists of a nucleotide sequence with sequence identity to a sequence comprising or consisting of SEQ ID NO: 1, wherein at least 500, 1000, 1500, 2000, 2500, 3000, 3500, 4000, 4500 or 5000 nucleotides corresponding to all or part of nucleotides 847 to 2753 and 34,220 to 36,443 have been deleted.

In another embodiment, the viral vector comprises or consists of a nucleotide sequence with sequence identity to a sequence comprising or consisting of SEQ ID NO: 1, wherein at least 500, 1000, 1500, 2000, 2500, 3000, 3500, 4000, 4500 or 5000 nucleotides corresponding to all or part of nucleotides 847 to 2753, 34,220 to 36,443, and 38,807 to 40,561 or 41,461 to 42398 have been deleted.

At least one exogenous nucleotide sequence is optionally inserted into the FAdV-9 viral vector described herein. Accordingly, in one embodiment, the FAdV-9 nucleotide sequence with the deletions described herein is not present in the vector as one contiguous sequence but rather includes sections of contiguous sequences interrupted by at least one, and optionally at least two, three or four, exogenous nucleotide sequences. Therefore, in one embodiment, the fowl adenovirus described herein comprises one or more nucleotide sequence(s) with sequence identity to the one or more sequences shown in SEQ ID NO: 1, wherein at least ORF17 has been deleted, and the nucleotide sequence comprises at least two, three, four or five contiguous sequences.

The number of nucleotides deleted from the FAdV-9 varies. In one embodiment, 1000 to 7000 nucleotides are deleted, optionally split between the left and the right end of the genome. In other embodiments, 1500 to 6000 nucleotides are deleted. In one embodiment, at least 500, 1000, 1500, 2000, 2500, 3000, 3500, 4000, 5000 or 6000 nucleotides are deleted.

The size of the exogenous nucleotide sequence(s) inserted into the viral vector described herein also varies. In one embodiment, based on the 105% adenovirus stability rule, the capacity of the vector is up to 7751 bp. In other embodiments, 1000 to 7000 nucleotides are inserted, optionally split between the left and the right end of the genome. For example, one foreign gene may be inserted into the left end and a second foreign gene may be inserted into the other end. In other embodiments, 1500 to 6000 nucleotides are inserted. In one embodiment, at least 1000, 2000, 3000, 4000, 5000 or 6000 exogenous nucleotides are inserted.

In one embodiment, the FAdV-9 viral vector further has a deletion at the left end of the genome of about 2291 base pairs, optionally between about 1900 and 2500 base pairs. In one embodiment, the FAdV-9 viral vector has a deletion at the right end of the genome of about 3591 base pairs. In one embodiment, the FAdV-9 viral vector has a deletion at the right end of the genome of between about 3000 base pairs and 4000 base pairs.

Sequence identity is typically assessed by the BLAST version 2.1 program advanced search (standard default parameters; Altschul, S.F., Gish, W., Miller, W., Myers, E.W. & Lipman, D.J. (1990) "Basic local alignment search tool." J. Mol. Biol. 215:403_410). BLAST is a series of programs that are available online through the U.S. National Center for Biotechnology Information (National Library of Medicine Building 38A Bethesda, MD 20894). The advanced Blast search is set to default parameters. References for the Blast Programs include: Altschul, S.F., Gish, W., Miller, W., Myers, E.W. & Lipman, D.J. (1990) "Basic local alignment search tool." J. Mol. Biol. 215:403-410; Gish, W. & States, D.J. (1993) "Identification of protein coding regions by database similarity search." Nature Genet. 3:266-272.; Madden, T.L., Tatusov, R.L. & Zhang, J. (1996) "Applications of network BLAST server" Meth. Enzymol. 266:131-141; Altschul, S.F., Madden, T.L., Schäffer, A.A., Zhang, J., Zhang, Z., Miller, W. & Lipman, D.J. (1997) "Gapped BLAST and PSI-BLAST: a new generation of protein database search programs." Nucleic Acids Res. 25:3389-3402; Zhang, J. & Madden, T.L. (1997) "PowerBLAST: A new network BLAST application for interactive or automated sequence analysis and annotation." Genome Res. 7:649-656.

As used herein, "viral vector" refers to a recombinant adenovirus that is capable of delivering an exogenous nucleotide sequence into a host cell. For example, in one embodiment, the viral vector comprises restriction sites that are suitable for inserting an exogenous nucleotide sequence into the vector. In one embodiment, one or more nucleotide sequences which are not required for the replication or transmission of FAdV-9 described herein are deleted in the nucleotide sequence of the viral vector. For example, in one embodiment nucleotide sequences at the left and right end of the FAdV-9 genome are deleted in the recombinant FAdV-9 viral vector. In one embodiment, in addition to the nucleotide sequence of ORF17 and/or ORF19, nucleotide sequences corresponding to one or more of ORFs 0-2, TR2, and ORF11 are deleted in the recombinant FAdV-9 viral vector. In one embodiment, the viral vector includes one or more exogenous control sequences such as promoters or cloning sites useful for driving the expression of transgenes. In one embodiment the promoters are selected from the group consisting of CMV (SEQ ID NO: 4), CAG (SEQ ID NO: 5), EF1α (SEQ ID NO: 6), β-actin (SEQ ID NO: 8) and L2R (SEQ ID NO: 7).

In one embodiment, the viral vector comprises an exogenous nucleotide sequence coding for a polypeptide of interest. In one embodiment, the polypeptide of interest is an antigen from a disease of concern. For example, in one embodiment, the viral vector comprises an exogenous nucleotide sequence coding for at least one antigenic site of a disease of concern. Exogenous nucleotide sequences coding for a polypeptide of interest can readily be obtained by methods known in the art such as by chemical synthesis, screening appropriate libraries or by recovering a gene sequence by polymerase chain reaction (PCR).

With respect to the present disclosure, diseases of concern include, but are not limited to, influenza, infectious laryngotracheitis (ILT), infectious bronchitis (IB), infectious bursal disease (Gumboro), hepatitis, viral rhinotracheitis, infectious coryza, Mycoplasma hyopneumoniae, pasteurellosis, Porcine Respiratory and Reproductive Syndrome (PRRS), circovirus, bordetellosis, parainfluenza, Avian influenza, Newcastle disease (NDV), infectious anemia, Inclusion bodies hepatitis (IBH), and Metapneumovirus (MPV).

In one embodiment, the viral vector is adapted to express an exogenous nucleotide sequence in a host cell. For example, in one embodiment the viral vector comprises control sequences capable of affecting the expression of an exogenous nucleotide sequence in a host. For example, the viral vectors described herein may include one or more control sequences such as a transcriptional promoter, an enhancer, an optional operator sequence to control transcription, a sequence encoding suitable mRNA ribosomal binding sites, alternative splicing sites, translational sequences, or sequences which control the termination of transcription and translation. Optionally, the viral vector comprises different control sequences at the left end and right end of the vectors. In one embodiment the promoters are selected from the group consisting of CMV (SEQ ID NO: 4), CAG (SEQ ID NO: 5), EF1α (SEQ ID NO: 6), β-actin (SEQ ID NO: 8) and L2R (SEQ ID NO: 7) and the enhancer may be WPRE (SEQ ID NO: 9).

In one embodiment, the viral vector comprises one or more exogenous nucleotide sequences operably linked to one or more control sequences. In one embodiment, the viral vector comprises an insertion site adjacent to one or more control sequences such that when an exogenous nucleotide sequence is inserted into the vector, the exogenous nucleotide sequence is operably linked to the control sequences. As used herein, nucleotide sequences are "operably linked" when they are functionally related to each other. For example, a promoter is operably linked to a coding sequence if it controls the transcription of the sequence; a ribosome binding site is operably linked to a coding sequence if it is positioned so as to permit translation. Optionally, sequences that are operably linked are contiguous sequences in the viral vector.

In one embodiment, the viral vector described herein includes a sequence suitable for the biological selection of hosts containing the viral vector such as a positive or negative selection gene.

Other methods known in the art, such as recombinant technologies including but not limited to those disclosed by Sambrook et al. (Sambrook J. et al. 2000. Molecular Cloning: A Laboratory Manual (Third Edition), Cold Spring Harbor Laboratory Press), are also suitable for preparing the nucleotide sequences and viral vectors as described herein.

Optionally, the viral vectors and methods described herein may be used for gene therapy in animal subjects in need thereof. For example, in one embodiment, the viral vectors described herein may be used for the delivery and expression of a therapeutic nucleotide sequence or nucleotide encoding a therapeutic protein. In one embodiment, there is provided a viral vector or composition as described herein for use in a method of gene therapy comprising administering to a subject in need thereof the viral vector or composition, wherein the viral vector comprises an exogenous nucleotide sequence encoding a therapeutic nucleotide sequence or protein.

Another aspect of the present disclosure includes an immunogenic composition comprising a recombinant FAdV-9 viral vector as described herein. In one embodiment, the immunogenic compositions can be prepared by known methods for the preparation of compositions for the administration to animals including, but not limited to, humans, livestock, poultry and/or fish. In one embodiment, an effective quantity of the viral vector described herein is combined in a mixture with a pharmaceutically acceptable carrier. Suitable carriers are described, for example in Remington's Pharmaceutical Sciences (Remington's Pharmaceutical Sciences, Mack Publishing Company, Easton, Pa., USA) or Handbook of Pharmaceutical Additives (compiled by Michael and Irene Ash, Gower Publishing Limited, Aldershot, England (1995)). On this basis, the compositions include, albeit not exclusively, solutions of the viral vectors described herein in association with one or more pharmaceutically acceptable carriers or diluents, and may be contained in buffered solutions with a suitable pH and/or be iso-osmotic with physiological fluids. In one embodiment, the immunogenic composition comprises an adjuvant.

The novel FAdV-9 viral vectors, associated methods and uses will be more clearly illustrated by means of the following description of specific examples.

### EXAMPLE 1

### Materials and Methods

### 1.1 Cell culture and viruses

Chicken hepatoma cells (CH-SAH cell line) were maintained in Dulbecco's Modified Eagle's Medium/Nutrient Mixture F-12 Ham (DMEM-F12) (Sigma) plus 200 mM L-glutamine and 100 U/ml penicillin-streptomycin (PenStrep, Sigma) with 10% non-heat inactivated fetal bovine serum (FBS) as described (Alexander, H.S., Huber, P., Cao, J., Krell, P.J., Nagy, É. 1998. Growth Characteristics of Fowl Adenovirus Type 8 in a Chicken Hepatoma Cell Line. J. Virol. Methods. 74, 9-14.). Recombinant FAdVs were generated using the FAdV-9Δ4 deletion virus described by Corredor and Nagy (2010b) as the base. Propagation of all viruses were carried out in CH-SAH cells as described by Alexander *et al.* (1998).

### 1.2 General DNA manipulation

### 1.2.1 Bacterial cultures and plasmid isolation

*Escherichia coli* DH5α cells were the bacterial host for all plasmids described, while *E. coli* BJ5183 cells were used for homologous recombination to generate recombinant FAdmids. Bacterial cultures were grown on selective Luria-Bertani (LB) liquid or agar (16 mg/ml) growth medium containing ampicillin (100 µg/ml) at 37°C. Single *E*. *coli* colonies were picked, inoculated in 5 ml of LB medium supplemented with 0.1% ampicillin to select for growth of bacteria containing a transformed ampicillin resistant plasmid. Incubation and growth was for approximately 16 hours. Chemically (CaCl₂) competent DH5α *E*. *coli* were prepared and transformed with either 10 µl of ligation product or 1 µl purified plasmid DNA (Sambrook, J., Russel, D.W. 2001. Molecular Cloning: A Laboratory Manual, Volume 1. 3rd Ed. Cold Spring Harbor Laboratory Press, Cold Spring, New York, USA). All plasmids were isolated using either the EZ-10 Spin Column Plasmid DNA Mini-prep kit (Bio Basic) or the PureLink HiPure Plasmid Midiprep kit (Invitrogen) (for plasmids larger than 40 kb in size or when a high concentration of DNA was needed) as per the manufacturer's protocol.

### 1.2.2 PCR and restriction enzyme digestion

DNA was amplified by polymerase chain reaction (PCR) during the cloning of all dual-expression constructs and recombinant viruses. For all cloning, PCR amplification was conducted using a *Kod* Hot Start Polymerase kit (Novagen). However, *Taq* polymerase was used when screening a plasmid by PCR. Unless stated otherwise, the PCR conditions for both *Kod* and *Taq* polymerases are summarized in Table 2. All PCR reactions were carried out with a Mastercycler Pro (Eppendorf).

Restriction enzyme (RE) digestions were carried out for both Fast Digest enzymes (Fermentas) and enzymes from New England BioLabs (NEB). All reactions occurred as per the manufacturer's protocol (per enzyme). Digestion reactions were always performed at 37°C, and samples were heat inactivated in a Mastercycler Pro (Eppendorf) thermocycler.

Unless otherwise stated, all DNA samples were subjected to electrophoresis at 100V in 0.8% agarose gels containing 1x RedSafe^{™} (iNtRON Biotechnology). 6X DNA loading buffer [0.25% (w/v) bromophenol blue, 40% sucrose (w/v) in water] and 1X Tris-acetate-EDTA (TAE) buffer were used for electrophoresis of DNA samples.

### 1.2.3 Plasmid construction and transformation

After RE digestion and gel purification, PCR amplified DNA and plasmid were ligated together with T4 DNA ligase (Invitrogen). Unless stated otherwise, ligations were performed at a molar ratio of 1:1 insert to vector, overnight at 16°C. Fifty µl of CaCl₂ competent DH5α *E*. *coli* were mixed with 10 µl of ligation product or 1 µl purified plasmid DNA. Competent cells and DNA were incubated on ice for 30 min, then heat shocked at 42°C for 1 min. Cells were recovered on ice for 3 min and 500 µl of super optimal broth with catabolite repression (SOC) medium was added to each microcentrifuge tube. The cells were incubated at 37°C for 1 hr with agitation, then centrifuged and resuspended in 100 µl of LB broth. The entire volume was spread on a LB agar plate containing ampicillin.

Homologous recombination occurred in *E. coli* BJ5183 cells. Two µg of both promoter cassettes and linear FAdV-9Δ4 DNA were mixed in 100 µl of chemically competent BJ5183 cells. Mixtures were left on ice for 15 min, followed by a heat shock at 42°C for 1 min. Cells were recovered on ice for 20 min, and 1 ml of SOC medium was added to each microcentrifuge tube and transferred into a 5 ml glass culture tube. Cells were incubated for 2 hrs at 37°C with agitation, then centrifuged and resuspended in 100 µl of LB broth. The entire volume of cells was spread onto a LB agar plate containing ampicillin.

### 1.2.4 Sequencing

All PCR products and plasmids were purified and sequenced by the ABI 3730 DNA sequencer (Laboratory Services Division, Guelph, ON). Sequence data were analysed using SnapGene Viewer (GSL Biotech).

### 1.3 Analysis of promoter expression

### 1.3.1 Generation of dual-expression plasmid constructs

The activity of five promoters (CMV, CAG, EF1α, β-actin, and L2R) and one enhancer element (WPRE) were compared by measuring the expression of EGFP compared to firefly luciferase under the SV40 promoter in transfected CH-SAH cells. The plasmids, pCl-Neo (Promega), pCAG-Puro, and pEF1α-Puro, were provided by Dr. Sarah Wootton (University of Guelph). Dual-expression plasmids were generated using the plasmid pCl-Neo as a backbone (Figure 11), which contained the CMV promoter along with numerous unique RE sites. Both the CAG and EF1α promoter were sub-cloned from pCAG-Puro and pEF1α-Puro, respectively, into pCl-Neo using Spel and EcoRI. The presence of each promoter was confirmed by sequencing using the primer pCl-Neo-F (Table 3). EGFP was amplified by PCR from pEGFP-N1 (Clontech Laboratories, Inc) with primers EGFP-F and EGFP-R (Table 3) at an annealing temperature of 60°C. The resulting PCR product was gel extracted using the Wizard Plus SV Miniprep DNA Purification Kit (Promega). Both EGFP PCR product and pCl-Neo based plasmids (containing the CMV, CAG, or EF1α promoter) were subjected to double digestion with EcoRI and Notl for 1 hr at 37°C. Both digested plasmid and PCR product were then separated in a gel and extracted using Wizard Plus SV Miniprep DNA Purification Kit (Promega), and ligated overnight at 4°C. Following transformation into E. coli DH5α cells and growth on LB-amp plates, colonies were PCR screened for the presence of the EGFP fragment with primers EGFP-F and EGFP-R. All positive colonies were confirmed by sequencing with EGFP-I-F primer (Table 3), resulting in the plasmids pCMV-EGFP, pCAG-EGFP, and pEF1α-EGFP.

The β-actin promoter was PCR amplified from pCAG-Puro using the primers βactin-F and βactin-R (Table 3) with an annealing temperature of 60°C. The resulting PCR product was gel extracted with the Wizard Plus SV Miniprep DNA Purification Kit (Promega). Both β-actin PCR product and pCAG-EGFP were subjected to double digestion with EcoRI and Notl for 1 hr at 37°C. Digested plasmid and PCR product were then separated in a gel and extracted using Wizard Plus SV Miniprep DNA Purification Kit (Promega), and ligated overnight at 4°C. Following transformation into *E*. coli DH5α cells and growth on LB-amp plates, colonies were screened with RE for the presence of β-actin. All positive colonies were confirmed by sequencing using both pCl-Neo-F and EGFP-I-F primers (Table 3), resulting in the plasmid pβactin-EGFP.

The fowlpox virus L2R promoter was PCR amplified from pE68 (Zantinge, J.L., Krell, P.J., Derbyshire, J.B., Nagy, É. 1996. Partial transcriptional mapping of the fowlpox virus genome and analysis of the EcoRI L fragment. J. Gen. Virol. 77(4), 603-614) with the primers L2R-F and L2R-R (Table 3) at an annealing temperature of 60°C. The resulting PCR product was gel extracted using the Wizard Plus SV Miniprep DNA Purification Kit (Promega). Both L2R PCR product and pCMV-EGFP were subjected to double digestion with EcoRI and Notl for 1 hr at 37°C. Both digested plasmid and PCR product were then separated in a gel and extracted using Wizard Plus SV Miniprep DNA Purification Kit (Promega), and ligated overnight at 4°C. Following transformation into E. coli DH5α cells and growth on LB-amp plates, colonies were screened with RE for the presence of L2R. All positive colonies were confirmed by sequencing with both pCl-Neo-F and EGFP-I-F primers (Table 3), resulting in the recovery of the plasmid pL2R-EGFP.

Firefly luciferase was PCR amplified from pGL4.17 (Promega) with primers Luc-F and Luc-R (Table 3) at an annealing temperature of 55°C. The resulting PCR product (1.6 kb) was gel extracted using the Wizard Plus SV Miniprep DNA Purification Kit (Promega). Both luciferase PCR product and promoter plasmids were subjected to double digestion with Avril and BstBI for 1 hr at 37°C. Digested plasmid and PCR product were then separated in a gel, removing the neomycin resistance (NeoR) cassette from each plasmid, and extracted using Wizard Plus SV Miniprep DNA Purification Kit (Promega), and ligated overnight at 4°C. Following transformation into E. coli DH5α cells and growth on LB-amp plates, colonies were PCR screened for the presence of luciferase. All positive colonies were confirmed by sequencing using SV40-F primer (Table 3).

Five additional dual-expression plasmids were generated to include the enhancer element WPRE. The WPRE element was PCR amplified from pWPRE (Dr. Sarah Wootton, University of Guelph) using the primers WPRE-F and WPRE-R (Table 2.2) with an annealing temperature of 55°C. The PCR product was gel extracted using the Wizard Plus SV Miniprep DNA Purification Kit (Promega). Both WPRE PCR product and promoter were subjected to digestion with Notl for 1 hr at 37°C. After digestion, plasmids were treated with alkaline phosphatase (calf intestinal, New England BioLabs) as per the manufacturer's protocol to prevent re-ligation. Both digested/dephosphorylated plasmid and PCR product were then separated in a gel and DNA extracted using Wizard Plus SV Miniprep DNA Purification Kit (Promega), and ligated overnight at 4°C. Following transformation into E. coli DH5α cells and growth on LB-amp plates, colonies were PCR screened for the presence of WPRE. All positive colonies were confirmed by sequencing using EGFP-I-F (Table 3). A list of all plasmids generated in this study and their purposes is in Table 4.

### 1.3.2 Transfection of chicken hepatoma cells

The expression of EGFP was measured by transfecting CH-SAH cells with the dual-expression plasmids. Cells were seeded in 35 mm dishes at a density of 1.2x10⁶ cells/dish and incubated at 37°C with 5% CO₂. Lipofectamine 2000 (Invitrogen) was used to transfect all constructs according to the manufacturer's recommendation. Briefly, 2 µg of dual-expression plasmid and 5 µl of Lipofectamine were incubated in separate 50 µl aliquots of Opti-Mem medium (Gibco) for 5 minutes, then mixed and incubated together for 20 minutes. During this period of time, the medium was removed from each dish, and the cell monolayers were washed two times with phosphate buffered saline (PBS). Two ml of DMEM-F12 (5% FBS) without antibiotics was added to each dish. After 20 minutes, the plasmid-lipofectamine mixtures were added to the 35 mm dishes and incubated for 6 hrs at 37°C in the presence of 5% CO₂. This was repeated for all ten dual-expression plasmids. After 6 hrs the medium was removed and fresh DMEM-F12 (5% FBS) was added. Every 12 hours post-transfection (h.p.t.), EGFP expression was confirmed by fluorescence microscopy and whole cell lysate was collected. Monolayers were washed with PBS, trypsin was added, and the cells were resuspended in DMEM-F12 (10% FBS). Cells were then centrifuged in 15 ml conical tubes (Nunc^{®}), the supernatant was removed, and the cell pellet was resuspended in 500 µl PBS and frozen at -80°C. Transfected cell samples frozen at -80°C were freeze-thawed three times. The cell debris was spun down at 12,000 rpm in a microcentrifuge for 10 minutes at 4°C. Supernatant was transferred to a fresh microcentrifuge tube and the protein concentration was determined at 280 nm using a Nanodrop 2000 (Thermo Scientific). All samples were adjusted to a protein concentration of 1 µg/µL.

### 1.3.3 Fluorescence microscopy

Both transfected and infected CH-SAH cells were monitored by fluorescence microscopy with a Zeiss fluorescence microscope (Carl Zeiss) with FITC optics.

### 1.3.4 Measuring reporter gene expression

The expression of EGFP was quantified by spectrofluorometry using a GloMax^{®}-Multi (Promega) microplate reader. Briefly, 50 µg of protein lysate was added in triplicate to a flat-bottomed black 96-well plate (Corning). Fluorescence of EGFP was measured in a GloMax^{®}-Multi (Promega) microplate reader at 480 nm excitation and 528 nm emission wavelengths. The three readings were averaged to give one fluorescence value per sample.

Luciferase expression was determined using a Pierce Firefly Luciferase Glow Assay kit (Thermo Fisher Scientific) as per the manufacturer's protocol. Briefly, 25 µg of protein lysate was added in triplicate to a flat-bottomed black 96-well plate (Corning). Fifty µl of Luciferase assay substrate was manually added to each replicate, mixed well, and incubated for 15 minutes protected from light before measuring luminescence in a GloMax^{®}-Multi (Promega) microplate reader. The readings were averaged to give one luminescence value per sample.

### 1.3.5 Normalizing promoter expression

Normalization of dual-expression constructs was performed to remove sample-to-sample variability of transfection efficiency (Schagat, T., Paguio, A., Kopish, K. 2007. Normalizing Genetic Reporter Assays: Approaches and Considerations for Increasing Consistency and Statistical Significance. Cell Notes. 17, 9-12). The fold change in activity was determined between promoter constructs and pCMV-EGFP-Luc. To begin, for each specific time-point and repetition the average fluorescence and luminescence value was determined for each plasmid construct, where a ratio of fluorescence over luminescence (F/R) was calculated. Activity level is being compared to the CMV promoter, currently used in recFAdVs, therefore the F/R ratio of CMV was set to a value of 1.0 (was divided by its own F/R ratio). Each remaining construct F/R value was also divided by the F/R value of CMV, resulting in a value representing the normalized fold change in activity (Δfold). Average fold change between repetitions was compared between all constructs at each time-point.

### 1.4 Generation and characterization of recombinant viruses

### 1.4.1 Construction of intermediate constructs

Further analysis of EGFP expression *in vitro* was performed with recFAdVs containing the CMV, CAG, and Ef1α based expression cassettes. In previous studies, recFAdVs were recovered by homologous recombination between pFAdV-9Δ4 and the PCR amplified expression cassette containing viral flanking regions, isolated from an intermediate construct. The plasmid pleftΔ491-2,782 (pLΔ2.4) contains the left end Δ4 deletion site (Δ491-2,782 nt) of FAdV-9 with a Swal RE site for blunt-cloning a transgene (Corredor and Nagy, 2010b). In this study, a new intermediate construct system was developed by cloning viral flanking regions directly into the dual-expression plasmids, thus creating plasmids ready for recombination (pHMR). Viral genomic regions flanking the Δ4 deletion site of FAdV-9 were PCR amplified from the intermediate construct pLΔ2.4. The region left of the deletion site (VF1) was PCR amplified using 100 ng of pLΔ2.4 and primers VF1-F and VF1-R (Table 3) with an annealing temperature of 52°C. The resulting PCR product was gel extracted using the EZ-10 Spin Column Plasmid DNA Mini-prep kit (Bio Basic). Both VF1 PCR product and all dual expression vectors were digested with Spel for 1 hr, followed by digestion with Bglll. Both digested plasmid and PCR product were then separated in a gel and extracted using QIAEX II Gel Extraction kit (Qiagen), and ligated overnight at 16°C. Following transformation into *E*. coli DH5α cells and growth on LB-amp plates, colonies were PCR screened for the presence of the VF1 fragment with primers VF1-F and VF1-R. All positive colonies were confirmed by sequencing. Next, the region right of the deletion site (VF2) was PCR amplified using 100 ng of pLΔ2.4 and primers VF2-F and VF2-R (Table 3) with an annealing temperature of 52°C. The resulting PCR product was gel extracted with the EZ-10 Spin Column Plasmid DNA Mini-prep kit (Bio Basic). Both VF2 PCR product and all dual expression vectors positive for the VF1 fragment were digested with Kpnl for 1 hr, followed by digestion with Mfel. Both digested plasmid and PCR product were then separated in a gel and extracted using QIAEX II Gel Extraction kit (Qiagen), and ligated overnight at 16°C. Following transformation into *E*. coli DH5α cells and growth on LB-amp plates, colonies were PCR screened for the presence of the VF2 fragment using VF1-F and VF2-R primers (Table 3) at an annealing temperature of 52°C, with an expected size of 2 kb plus the size of each EGFP expression cassette. A list of all six pHMR intermediate plasmids is in Table 4.

### 1.4.2 Generating recombinant fowl adenoviruses

Recombinant FAdVs were generated to include the CMV, CMV-WPRE, CAG, CAG-WPRE, EF1α, and EF1α-WPRE expression cassettes using a method modified from Corredor and Nagy (2010b) (Figure 12). Expression cassettes flanked by viral DNA in the pHMR plasmids were recombined with pFAdV-9Δ4 to create new recombinant FAdmids, containing a promoter of interest and EGFP. To obtain promoter EGFP cassettes flanked by viral DNA sequences, intermediate pHMR constructs were subjected to PCR or RE digestion with BgIII/BamHI. Cassettes containing the CMV promoter (pHMR-CMV-EGFP and pHMR-CMV-EGFP-WPRE) and EF1α promoter (pHMR- EF1α-EGFP and pHMR- EF1α-EGFP-WPRE) were PCR amplified. PCR was carried out with 200 ng of plasmid and primers E1-F and E1-R (Table 3) with an annealing temperature of 52°C, and the resulting PCR product was gel extracted using QIAEX II Gel Extraction kit (Qiagen). Cassettes containing the CAG promoter (pHMR-CAG-EGFP and pHMR-CAG-EGFP-WPRE) were double-digested using BgIII/BamHI. Briefly, 5 µg of plasmid was digested with both enzymes for 1 hr at 37°C. Samples were separated by gel electrophoresis, and bands corresponding to CAG cassette (4.5 kb) or CAG-WPRE cassette (5 kb) were gel extracted using EZ-10 Spin Column DNA Gel Extraction kit (Bio Basic). Next, 5 µg of pFAdV-9Δ4 was linearized with Swal and ethanol precipitated. The concentration of all DNA obtained by PCR and RE digestion product was measured with a Nanodrop 2000 (Thermo Scientific). For each construct, two µg of promoter EGFP cassette and 2 µg of Swal digested pFAdV-9Δ4 were co-transformed into E. coli BJ5183 cells. All resulting constructs were screened for recombination by Notl digestion followed by transformation into E. coli DH5α. These constructs were grown up in LB medium, isolated using PureLink HiPure Plasmid Midiprep kit (Invitrogen), digested with Pad, and extracted by ethanol precipitation. CH-SAH cells were seeded into 25 cm flasks at a density of 4.3×10⁶ cells/flask. Five µg of Pacl digested DNA was transfected into the CH-SAH cells using 25 µl of Lipofectamine 2000 (Invitrogen). After 6 hrs the transfection mixture was removed and fresh DMEM-F12 (5% FBS) was added, the cells were incubated at 37°C with 5% CO₂. Cells were monitored for the appearance of cytopathic effect (CPE) for the next week. At this point cell cultures were frozen and thawed three times, centrifuged, and the clarified supernatant containing recombinant FAdV-9Δ4 was stored as P0 stock at -80°C. Virus was subsequently passaged three times to obtain a high titre P4 stock. A list of recFAdVs is in Table 4.

### 1.4.3 Viral growth curves

One-step growth curves for all viruses were obtained as described by Alexander *et al.* (1998). Briefly, a total of 1.8×10⁶ CH-SAH cells were seeded in 35 mm dishes and incubated at 37°C with 5% CO₂. The cells were infected with recFAdVs at a multiplicity of infection (MOI) of 5. After adsorption for 1 hour at room temperature, the cells were washed three times with PBS, and fresh DMEM-F12 (5% FBS) was added. Cell culture medium and cells were harvested at 0, 12, 18, 24, 30, 36, 48, 60, and 72 hours post-infection (h.p.i.). The medium was removed and frozen at -80°C as the extracellular virus, while the cells were washed three times with PBS, 1 ml of medium was placed on the monolayer and the dish frozen at -80°C as the intracellular virus. Extracellular virus was titrated for each time-point. CH-SAH cells were plated in 6-well plates at a density of 1.8×10⁶ cells/well and incubated overnight. Extracellular virus from each time-point was serially diluted (10⁻¹-10⁻⁷), and 100 µl of each aliquot was inoculated in duplicate and allowed to adsorb for 1 hr at room temperature. The inoculum was removed and the monolayer was washed in PBS. Three ml of agar layer consisting of 0.6% SeaKem LE agarose (Lonza), DMEM-F12 (5% FBS, L-glutamine, and PenStrep) was added to each well, and the plates were incubated at 37°C with 5% CO₂. After five days, 1.5 ml of neutral red (0.015%) was added to each well, and after 24 hrs the plaques were counted.

### 1.4.4 Detection of EGFP expression

A total of 1.8×10⁶ CH-SAH cells were seeded in 35 mm dishes and incubated at 37°C with 5% CO₂. The cells were infected with recFAdVs at an MOI of 5. Uninfected and FAdV-9Δ4 infected cells were the negative controls, while cells transfected with 6 µg of pEGFP-N1 were the positive control. Cells were collected at 0, 6, 12, 18, 24, 30, 36, and 48 h.p.i. and centrifuged at 5,000 rpm for 5 minutes. Each sample was resuspended in 500 µl of PBS and split into two 250 µl aliquots. The first aliquot was stored frozen at -80°C to later be measured by spectrofluorometry. The second aliquot was centrifuged again to wash away FBS. The supernatant was removed and the cell pellet was resuspended in 200 µl of RIPA lysis buffer (50 mM Tris HCl pH 7.5, 150 mM NaCl, 1% Triton X-100, 0.1% SDS, 10 mM EDTA, and 1% sodium deoxycholate). Samples were incubated on ice for 20 minutes, and re-centrifuged at 12,000 rpm at 4°C for 20 minutes. The supernatant was collected and stored at -80°C.

EGFP expression was measured by spectrofluorometry at each time-point. Infected cell samples frozen at -80°C were freeze-thawed three times. The cell debris was spun down at 12,000 rpm for 10 minutes at 4°C. Supernatant was transferred to a fresh microcentrifuge tube and the protein concentration was determined at 280 nm using a Nanodrop 2000 (Thermo Scientific). All samples were adjusted to a protein concentration of 1 µg/µL. For each sample, 50 µg of protein extract was added in triplicate to a flat-bottomed black 96-well plate (Corning) and analyzed using a GloMax^{®}-Multi (Promega) microplate reader to detect EGFP fluorescence using 480 and 528 nm excitation and emission wavelengths, respectively.

### 1.4.5 Bradford assay and SDS-PAGE

Protein concentration was determined with the BioRad Protein Assay kit as per the manufacturer's protocol. Briefly, a 1:5 dilution of concentrated dye reagent was made in distilled H₂O. Ten µl of BSA protein standards, ranging in concentration from 100 µg/ml to 1 mg/ml, along with whole cell lysate samples (diluted 1:10) were pipetted into a 96-well plate in triplicate. Two hundred µl of the diluted dye reagent was added to each sample and mixed. After a 5 minute incubation, absorbance was measured at 595 nm in a microplate reader (BioTek Powerwave XS2). A standard curve was generated using the absorbance values of the BSA standards, and the equation of the trend line was used to extrapolate the concentrations of the unknown cell lysates. Values for each sample were averaged to generate an average total protein concentration, and dilution factor were accounted for. All samples were diluted to a final concentration of 0.67 µg/µL in distilled H₂O.

Proteins were separated via SDS-polyacrylamide gel electrophoresis (SDS-PAGE). Ten percent acrylamide gels were prepared according to recipes obtained from the Roche Lab FAQs Handbook (4^{th} Ed). For all experiments, 15 µl of each cell lysate (0.67 µg/µL) was mixed with 4 µl of 4x SDS-PAGE loading buffer (supplemented with 2-mercaptoethanol). Samples were incubated at 95°C for 10 minutes prior to being loaded in the gels. A total of 10 µg was loaded per sample into individual lanes, as well as 5 µL of protein ladder (Precision Plus Protein Dual Colour, BioRad). Once all samples were loaded, gels were run at 100 V for approximately 1.5 hours in running buffer (25 mM Tris, 190 mM glycine, 0.1% SDS, pH 8.3).

### 1.4.6 Western immunoblot

Following SDS-PAGE, proteins were transferred onto polyvinylidene difluoride (PVDF) membranes in a Mini Trans-Blot Electrophoretic Transfer Cell (BioRad) as per the manufacturer's protocol. Samples were run at 100 V for 1 hour in transfer buffer (25 mM Tris, 190 mM glycine, 20% methanol, pH 8.3). After transfer, membranes were rinsed in Tris-buffered saline supplemented with 0.1% Tween 20 (TBS-T) and blocked with 5% skim milk (in TBS-T) for 1 hour at room temperature with agitation. Primary antibody was added to the blocking solution and membranes were incubated overnight at 4°C. To probe for EGFP, primary monoclonal mouse anti-GFP antibody (Molecular Probes) was used at a dilution of 1:1,000. To probe for actin, primary polyclonal goat anti-actin antibody (Santa Cruz Biotechnology) was used at a dilution of 1:1,000. The following day, all blots were washed three times in TBS-T for 10 minutes each with agitation. The blots were then incubated in secondary antibody diluted in blocking solution for 1 hour with agitation. The following polyclonal secondary antibodies conjugated with horseradish peroxidase (HRP) were used: goat anti-mouse (Invitrogen, at 1:5,000 dilution), and donkey anti-goat (Jackson, at 1:20,000 dilution). Blots were then washed three more times in TBS-T, and incubated in Western Lightning Plus-ECL reagent (Perkin Elmer Inc) for five minutes before being developed using a ChemiDoc XRS (BioRad).

**Table 2. PCR conditions using either Kod or Taq polymerase as per the manufacture's protocol.**

| ***Kod* Polymerase** | | | | |
|---|---|---|---|---|
| Step | Target size | | | |
| | <500 bp | 500-1000 bp | 1000-3000 bp | >3000 bp |
| Activation | 95°C for 2 min | | | |
| Denaturation | 95°C for 20 sec | | | |
| Annealing | Lowest Primer Tm°C for 10 sec | | | |
| Extension | 70°C for 10s/kb | 70°C for 15s/kb | 70°C for 20s/kb | 70°C for 25s/kb |
| Repeat Steps 2-4 | 25 cycles | | | |

### Taq Polymerase

| Step | |
|---|---|
| Activation | 95°C for 5 min |
| Denaturation | 95°C for 1 min |
| Annealing | Lowest Primer Tm°C for 30 sec |
| Extension | 70°C for 1 min/kb |
| Repeat Steps 2-4 | 25 cycles |

**Table 3. Primers designed for the generation of dual expression constructs and reFAdVs. Restriction enzymes incorporated into each primer are underlined.**

| Primer | Sequence (5' to 3') | Tm | Restriction Enzyme |
|---|---|---|---|
| pCl-Neo-F | GGCTCATGTCCAATATGACCGCCAT (SEQ ID NO: 22) | 61 °C | - |
| EGFP-F | AAAAAAGAATTCACCATGGTGAGCAAGGGCGAG (SEQ ID NO: 23) | 58 °C | EcoRI |
| EGFP-R | AAAAAAGCGGCCGCTTACTTGTACAGCTCGTCCATGCC (SEQ ID NO: 24) | 59 °C | Notl |
| EGFP-I-F | GAGAAGCGCGATCACATGGT (SEQ ID NO: 25) | 58 °C | - |
| pactin-F | AAAAAAACTAGTTGGTCGAGGTGAGCCCCACGTT (SEQ ID NO: 26) | 65 °C | Spel |
| βactin-R | AAAAAAGAATTCGCCCGCCGCGCGCTTCGCTT (SEQ ID NO: 27) | 71 °C | EcoRI |
| L2R-F | AAAAAAACTAGTCATAGTAAATATGGGTAACTTCTTAATAGCCA (SEQ ID NO: 28) | 55 °C | Spel |
| L2R-R | AAAAAAGAATTCATGGTTTGTTATTGGCAAATTTAAATTAATGTT (SEQ ID NO: 29) | 55 °C | EcoRI |
| Luc-F | AAAAAACCTAGGTTGGCAATCCGGTACTGTTGGT (SEQ ID NO: 30) | 59 °C | AvrII |
| Luc-R | AAAAAATTCGAAGCCGCCCCGACTCTAG (SEQ ID NO: 31) | 58 °C | BstBI |
| SV40-F | GGCCTCTGAGCTATTCCAGA (SEQ ID NO: 32) | 56 °C | - |
| WPRE-F | AAAAAAGCGGCCGCTCAACCTCTGGATTACAAAATTTGTGAA (SEQ ID NO: 33) | 56 °C | Notl |
| WPRE-R | AAAAAAGCGGCCGCGCCCAAAGGGAGATCCGAC (SEQ ID NO: 34) | 58 °C | Notl |
| VF1-F | AAAAAAAGATCTTACATGAATGACGCTGCTG (SEQ ID NO: 35) | 53 °C | BgIII |
| VF1-R | AAAAAAACTAGTAAATACACCGAGAAATACCACG (SEQ ID NO:36) | 53 °C | Spel |
| VF2-F | AAAAAACAATTGAAATAAAGACTCAGAACGCATTTTCC (SEQ ID NO: 37) | 54 °C | Mfel |
| VF2-R | AAAAAAGGTACCCCCCCGCAGAGAATTAAAA (SEQ ID NO: 38) | 53 °C | Kpnl |
| E1-F | ACATGAATGACGCTGCTG (SEQ ID NO: 39) | 53 °C | - |
| E1-R | CCCCCGCAGAGAATTAAAA (SEQ ID NO: 40) | 52 °C | - |

**Table 4. Vectors and viruses in this study and their key features.**

| | | |
|---|---|---|
| pHMR-CAG-EGFP-WPRE | 9,484 | Intermediate construct for homologous recombination with pFAdV-9Δ4 |
| pHMR-EF1α-EGFP | 8,766 | Intermediate construct for homologous recombination with pFAdV-9Δ4 |
| pHMR-EF1α-EGFP-WPRE | 9,290 | Intermediate construct for homologous recombination with pFAdV-9Δ4 |
| pFAdV-9Δ4 | 44,971 | FAdmid backbone Δ491-2,782 |
| pFAdV-9Δ4-CMV-EGFP | 46,826 | FAdmid containing CMV-EGFP in Δ4 site |
| pFAdV-9Δ4-CMV-EGFP-WPRE | 47,350 | FAdmid containing CMV-EGFP-WPRE in Δ4 site |
| pFAdV-9Δ4-CAG-EGFP | 47,583 | FAdmid containing CAG-EGFP in Δ4 site |
| pFAdV-9Δ4-CAG-EGFP-WPRE | 48,107 | FAdmid containing CAG-EGFP-WPRE in Δ4 site |
| pFAdV-9Δ4-EF1α-EGFP | 47,389 | FAdmid containing EF1α -EGFP in Δ4 site |
| pFAdV-9Δ4-EF1α-EGFP-WPRE | 47,913 | FAdmid containing EF1α-EGFP-WPRE in Δ4 site |

| **Viruses** | | |
|---|---|---|
| | | |
| Name | Size (bp) | Description |
| FAdV-9Δ4 | 42,772 | FAdV-9 deletion virus (Δ491-2,782) |
| FAdV-9Δ4-CMV-EGFP | 44,627 | Recombinant CMV-EGFP virus |
| FAdV-9Δ4-CMV-EGFP-WPRE | 45,151 | Recombinant CMV-EGFP-WPRE virus |
| FAdV-9Δ4-CAG-EGFP | 45,384 | Recombinant CAG-EGFP virus |
| FAdV-9Δ4-CAG-EGFP-WPRE | 45,908 | Recombinant CAG-EGFP-WPRE virus |
| FAdV-9Δ4-EF1α-EGFP | 45,190 | Recombinant EF1α-EGFP virus |
| FAdV-9Δ4-EF1α-EGFP-WPRE | 45,714 | Recombinant EF1α-EGFP-WPRE virus |

### EXAMPLE 2

### Generation and characterization of FAdV-9 viral vectors

As shown in Figures 1-8, the inventors performed experiments to generate and characterize FAdV-9 viral vectors including ORF0-ORF1-ORF2 deleted viruses with an inserted mCherry coding sequence and using the native early promoter; TR2-ORF17-ORF11 deleted viruses with an inserted EGFP cassette and a dual expression virus useful as a polyvalent vector.

At the left end of the genome: from nucleotide 847 to 2753; 1906 nucleotides were deleted. This deletion includes ORF1A, ORF1B, ORF1C, and ORF2. At the right end of the genome: from nucleotide 38,807 to 42,398; 3591 nucleotides were deleted. This deletion includes TR-2, ORF 17 and ORF 11. The total deletion is 5497 bp; the size of the dual deletion vector is 39,567 bp. The foreign gene inserted to left end was mCherry (SEQ ID NO: 3, 711 bp). The foreign gene inserted to right end was EGFP (SEQ ID NO: 2, 1602 bp). Based on the 105% adenovirus stability rule, the capacity of dual vector is up to 7751 bp, this could be in different configurations, e.g. at the left end is up to 4160 bp and at the right end is up to 5844 bp.

As shown in Figures 2 and 3A, at the left end of the genome: from nucleotide 575-2753: 2178 pb nucleotides were deleted. This deletion includes ORFO, ORF1A, ORF1B, ORF1C and ORF2 and the foreign gene inserted to the left end was mCherry (SEQ ID NO: 3, 711 pb). The verification of FAdmid and the corresponding viruses were performed (Figure 3B and 3D) which show that the construction was correct. On the other hand, a CPE of the recombinant viruses was observed on CH-SAH cells, however the infected cells did not show any mCherry fluorescence which indicates that the foreign gene, in this case, mCherry, is not being expressed (Figure 3C).

As shown in Figures 2 and 4A, at the left end of the genome: from nucleotide 847 to 2753; 1906 nucleotides were deleted. This deletion includes ORF1A, ORF1B, ORF1C, and ORF2. The foreign gene inserted to the left end was mCherry (SEQ ID NO: 3, 711 pb). The verification of FAdmid and the corresponding viruses were performed (Figure 4B and 4D) which show that the construction was correct. CH-SAH cells infected with the recombinant viruses showed mCherry fluorescence which indicates that the foreign gene, in this case, mCherry, is being expressed (Figure 4C).

As shown in Figures 5 and 7A, at the right end of the genome: from nucleotide 38,807 to 42,398; 3591 nucleotides were deleted. This deletion includes TR-2, ORF 17 and ORF 11. The foreign gene inserted to left end was EGFP (SEQ ID NO: 2 1602 bp). The verification of FAdmid and the corresponding viruses were performed (Figure 7B and 7D) which show that the construction was correct. CH-SAH cells infected with the recombinant viruses showed EGFP fluorescence which indicates that the foreign gene, in this case, EGFP, is being expressed (Figure 7C).

As shown in Figure 8A, at the left end of the genome: from nucleotide 847 to 2753; 1906 nucleotides were deleted. This deletion includes ORF1A, ORF1B, ORF1C, and ORF2. At the right end of the genome: from nucleotide 38,807 to 42,398; 3591 nucleotides were deleted. This deletion includes TR-2, ORF 17 and ORF 11. The foreign gene inserted to left end was mCherry (SEQ ID NO: 3, 711 bp). The foreign gene inserted to right end was EGFP (SEQ ID NO: 2, 1602 bp). The verification of FAdmid and the corresponding viruses were performed (Figure 8B and 8D) which show that the construction was correct. CH-SAH cells infected with the recombinant viruses showed mCherry and EGFP fluorescence which indicates that the foreign genes are expressed (Figure 8C).

Figure 6 shows an additional vector based on the deletion of ORF17 located at the right end of the genome of FAdV-9 as part of the logical and systematic development of the vector system.

From the above, it can be observed that when ORF0 is deleted the native early promoter is not functioning since no foreign gene expression is observed (e.g. m-Cherry). However, the expression of genes reporter does occur when exogenous (foreign) promotor (CMV) is used (Corredor and Nagy, 2010b). When only ORF1 and ORF2 are deleted the native promoter works and the foreign gene reporter expression is observed.

Figure 9 shows FadV-9 based recombinant viruses with reporter genes. Specifically, this figure is a summary and linear representation of Figures 4, 6, 7 and 8, wherein line pFAdV-9-RED represents Figure 4, line pFAdV-9-Δ17 represents Figure 6, line pFAdV-9-EGFP represents Figure 7, line pFAdV-9-Dual represents Figure 8.

Accordingly, the inventors have successfully replaced ORFs 0-2, ORFs 1-2, and TR2-ORFs 17-11 of FAdV-9 with reporter genes. It was observed that FAdV left end early native promoter can drive foreign transgene expression. Furthermore, a large right end deletion in TR2 was demonstrated to be stable, contrary to previous studies. FAdV-9 with deletions at the left end and right end of FAdV-9 therefore may be used as polyvalent recombinant FAdV-9 viruses and may have applications for creating dual expression viruses suitable for vaccine vectors.

The primers used in this study are disclosed in Table 5.

**Table 5.**

| Name | Sequence (5'-3') | Location | Purpose |
|---|---|---|---|
| ORF1-2Ver-F | gcacagtcccaatggctt (SEQ ID NO: 42) | Pei et al.,2015 | |
| ORF1-2Ver-R | aaattctggtccgttaccga (SEQ ID NO: 43) | Pei et al.,2015 | |
| TR2-17-11VerF | GCCTACTCCTCAGCCTATCAC (SEQ ID NO: 44) | 38163-38184 | verification |
| TR2-17-11VerR | CAGTCCTCTAACGAGCACGC (SEQ ID NO: 45) | 43113-43133 | verification |
| CAT-F | GTGTAGGCTGGAGCTGCTTC (SEQ ID NO: 46) | | Verification |
| ORF1CATSwal-F | ggacgtgctttaggtaatttcctccg (SEQ ID NO: 47) | 796-846 | Deleted ORF1, 1A, 1B, 1C, RF20 introduce Swal |
| | Ttctttttcccgtttaggtgaggag(SEQ ID NO: 48) | | |
| | ATTTAAAT**gtgtaggctggagctgcttc** (SEQ ID NO: 49) | | |
| ORF2CATSwal-R | gcgttctgagtctttattggtaa- (SEQ ID NO: 50) | Pei et al.,2015 | |
| | actcgaaacacgcgtcacacgcgc- (SEQ ID NO: 51) | | |
| | ATTTAAATcatatgaatatcctccttagttc (SEQ ID NO: 52) | | |
| TR2-17-11CAT-F | ccccctggcggccgttggccgccactg (SEQ ID NO: 53) | 38757-38806 | Deleted TR2, ORF17, ORF11 introduce Swal |
| | Cacccgcgccggacttagtctct (SEQ ID NO: 54) | | |
| | TTTAAAT**gtgtaggctggagctgcttc** (SEQ ID NO: 55) | | |
| TR2-17-11CAT-R | agtagagattataggaagcggggatta (SEQ ID NO: 56) | 42399-42448 | |
| | Tagtggtttttatttaaacgaga (SEQ ID NO: 57) | | |
| | ATTTAAAT**catatgaatatcctccttagttc** (SEQ ID NO: 58) | | |
| ORF17CATSwal-F | tccccctggcggccgagggccgcca (SEQ ID NO: 59) | 40511-40561 | Delete ORF17 |
| | Ctgcacccgcgccggacttagtctct (SEQ ID NO: 60) | | |
| | ATTTAAAT**gtgtaggctggagctgcttc** (SEQ ID NO: 61) | | |
| ORF17CATSwal-R | gatacgaagaaggataggagcagaat (SEQ ID NO: 62) | 41461-41502 | |
| | Cgaggatacggtagttgactcc (SEQ ID NO: 63) | | |
| | ATATTTAAAT**catatgaatatcctccttagttc** (SEQ ID NO: 64) | | |
| EGFPcaSwal-F | *agctgc*ATTTAAATgtattaccgccatgcattag (SEQ ID NO: 65) | Pei et al., 2015 | |
| EGFPcaSwal-R | *agctgc*ATTTAAATccacaactagaatgcagtg (SEQ ID NO: 66) | Pei et al., 2015 | |
| mCherry-F | *agctgcATTTAAAT*ATGGTGAGCAAGGGCGAGGAGG (SEQ ID NO: 67) | ??? | amplify mCherry |
| mCherry-R | *agctgcATTTAAAT*CTACTTGTACAGCTCGTCCATGCCG (SEQ ID NO: 68) | ??? | coding sequence |

| | | | |
|---|---|---|---|
| Swal sites are capitalized Sequences in bold are chloramphenicol cassette specific Underlined sequences are pN1-EGFP specific, the location is based on pEGFP-N1 Italicized sequences are extra nucleotides for Swal digestion | | | |

### EXAMPLE 3

### Results

### 3.1 Expression of EGFP in transfected CH-SAH Cells

### 3.1.1 Cloning dual-expression constructs

A dual-expression system was created using EGFP and firefly luciferase (expressed under the SV40 promoter) to compare the strength of different promoter and enhancer elements on EGFP expression *in vitro.* The commercial plasmid pCl-Neo (Promega), containing the CMV promoter, was the backbone for the dual-expression system. The CMV promoter (944 bp) was subsequently removed using RE digestion with Spel and EcoRI. After gel purification, both CAG (1,701 bp) and EF1α (1,507 bp) promoters were directionally sub-cloned into the pCI-Neo backbone using Spel and EcoRI. Ligated product was transformed into competent bacterial cells and colonies were selected and screened by RE digestion (results not shown) and confirmed by sequencing. This process was repeated with both the β-actin (285 bp) and L2R promoters (120 bp). The RE sites for Spel and EcoRI were inserted into primers and both promoters were PCR amplified from plasmid DNA.

A 730 bp band corresponding to EGFP was PCR amplified with primers containing EcoRI and Notl sites. PCR product was directionally cloned into each promoter plasmid, transformed into competent bacterial cells and confirmed by both PCR (data not shown) and sequencing. Finally, firefly luciferase (1,712 bp) was PCR amplified with primers containing the RE sites Avril and BstBI. Plasmid DNA, containing EGFP under the control of each promoter, was then digested with Avril and BstBI to remove the neomycin resistance gene, and luciferase was directionally cloned in its place. The presence of luciferase in each plasmid was confirmed by PCR (results not shown) and sequencing. This resulted in the dual-expression plasmids: pCMV-EGFP-Luc, pCAG-EGFP-Luc, pEF1α-EGFP-Luc, pβactin-EGFP-Luc, and pL2R-EGFP-Luc (Figure 11). Five additional plasmids were generated to include the enhancer element WPRE. A 543 bp band corresponding to WPRE was PCR amplified with primers containing a Notl site. Each dual-expression plasmid was linearized with Notl and the WPRE element was non-directionally cloned into each. Proper orientation was screened by PCR (data not shown) and plasmids were confirmed by sequencing, resulting in the dual-expression plasmids: pCMV-EGFP-WPRE-Luc, pCAG-EGFP-WPRE-Luc, pEF1a-EGFP-WPRE-Luc, pβactin-EGFP-WPRE-Luc, and pL2R-EGFP-WPRE-Luc (Figure 13).

### 3.1.2 Expression patterns of promoters observed by fluorescence microscopy

CH-SAH cells were transfected with the dual-expression constructs to follow EGFP expression patterns over 72 h.p.t. by fluorescence microscopy (Figure 14). The earliest expression of EGFP was noted at 12 h.p.t. by CMV, CMV-WPRE, CAG, and CAG-WPRE plasmids, with expression levels increasing over-time. Both EF1α and EF1α-WPRE constructs began expressing EGFP between 24 and 36 h.p.t. and remained steady in expression over-time. Constructs containing the β-actin or L2R promoters expressed EGFP the weakest in CH-SAH cells, with expression starting at 36 and 60 h.p.t., respectively. All constructs were compared to mock transfected cells (negative control) and a positive control of pEGFP-N1 transfected cells (data not shown).

### 3.1.3 Normalized expression of promoter constructs

Dual-expression constructs were transfected into CH-SAH cells and transgene expression was measured over 72 h.p.t. The fluorescence of EGFP was measured by fluorometry at each time-point, while the luminescence from luciferase was measured using a Peirce Firefly Luciferase Glow Assay kit (Thermo Fisher Scientific). To better analyse the expression of EGFP driven by each promoter/enhancer element, and to minimize sample-to-sample variation, the expression of luciferase from each sample was used to normalize the results (Schagat *et al.,* 2007). The data was analysed by calculating the fold change of each construct, at each specific time post-transfection, in relation to the CMV promoter (Table 6). The normalized expression of each construct from 12-72 h.p.t. is shown in Figure 15. At 12 h.p.t. significant decrease in expression was observed by EF1α, EF1α-WPRE, β-actin-WPRE, and L2R-WPRE constructs compared to CMV. CMV-WPRE and CAG constructs had increased expression at 12 h.p.t. however this was not significant. Greater differences in expression patterns were observed between 24-72 h.p.t. In general, all constructs showed decreased activity over-time compared to the CMV control. At both 12 and 24 h.p.t. the CMV-WPRE construct showed an increased fold change of 1.047 over CMV only, though the differences were not significant. Between 36 and 60 h.p.t. the CMV-WPRE showed a slightly decreased fold change ranging from 0.851 to 0.922. A significant decrease in expression was deleted at 72 h.p.t., when CMV-WPRE showed a lower activity level of 64% compared to the CMV control, and this difference was significant. For each of the remaining constructs, expression was significantly below that of CMV at all time-points where P <0.05. Constructs containing the CAG and CAG-WPRE cassettes consistently expressed at a range of approximately 50% and 40%, respectively, compared to CMV from 24 h.p.t. Similarly, constructs containing the EF1α and EF1α-WPRE cassettes were found to have a decreased activity level compared to CMV, expressing at a range of approximately 20%. The remaining constructs, containing the β-actin and L2R promoters, had activity levels less than 10% that of CMV.

### 3.2 Generation of recombinant FAdV-9Δ4s

### 3.2.1 Cloning pHMR constructs

To further analyse promoter activity and the activity of WPRE in the context of FAdV replication, recombinant FAdVs containing the most efficient EGFP expression cassettes were generated following a method modified from Corredor and Nagy (2010b). A 477 bp fragment (VF1) left of the FAdV-9Δ4 deletion site was PCR amplified and directionally cloned into pCMV-EGFP-Luc, pCMV-EGFP-WPRE-Luc, pCAG-EGFP-Luc, pCAG-EGFP-WPRE-Luc, pEF1α-EGFP-Luc, and pEF1α-EGFP-WPRE-Luc. Subsequently, a 1609 bp fragment (VF2) right of the FAdV-9Δ4 deletion site was PCR amplified and cloned into each plasmid, resulting in the intermediate plasmids: pHMR-CMV-EGFP, pHMR-CMV-EGFP-WPRE, pHMR-CAG-EGFP, pHMR-CAG-EGFP-WPRE, pHMR- EF1α-EGFP and pHMR- EF1α-EGFP-WPRE (Figure 16). Successful cloning was determined by PCR screening and sequencing.

Intermediate constructs were subjected to PCR or RE digestion with BgIII/BamHI to isolate promoter EGFP cassettes flanked by viral DNA fragments for use in homologous recombination with pFAdV-9Δ4 (FAdmid). The resulting FAdmids were generated upon homologous recombination: pFAdV-9Δ4-CMV-EGFP, pFAdV-9Δ4-CMV-EGFP-WPRE, pFAdV-9Δ4-CAG-EGFP, pFAdV-9Δ4-CAG-EGFP-WPRE, pFAdV-9Δ4-EF1α-EGFP, and pFAdV-9Δ4-EF1α-EGFP-WPRE. Successful recombination was determined by sequencing and Notl digest screening (Figure 17). Digested pFAdV-9Δ4 resulted in a 4 kb, 5.1 kb, 13.7 kb, and 23.8 kb band. FAdmids containing the CMV cassettes were identified by a diagnostic 2.2 kb band corresponding to the CMV promoter plus the EGFP gene and SV40 polyA tail. FAdmids containing the CAG cassettes were identified by a diagnostic 2.9 kb band, and EF1α by a diagnostic 2.7 kb band. Additional bands at 550 bp were present in FAdmids containing the WPRE element. These FAdmids were transfected into CH-SAH cells and to generate the corresponding viruses.

### 3.2.2 Viral growth kinetics

Viral growth kinetics were compared among the six recombinant viruses and the reference FAdV-9Δ4 to determine whether the addition of any promoter EGFP cassette affected virus replication and growth. The virus titer and growth of all recFAdVs appeared to follow a similar pattern to FAdV-9Δ4, except that FAdV-9Δ4-CAG-EGFP and FAdV-9Δ4-EF1α-EGFP grew to a titer one half log lower than the reference starting at 24 h.p.i. (Figure 18). Viruses grew to the following titers; FAdV-9Δ4 = 2.4×10⁸ pfu/ml, FAdV-9Δ4-CMV-EGFP = 4.8×10⁸ pfu/ml, FAdV-9Δ4-CAG-EGFP = 8.3×10⁷ pfu/ml, FAdV-9Δ4- EF1α-EGFP = 8.2×10⁷ pfu/ml, FAdV-9Δ4-CMV-EGFP-WPRE = 3.1×10⁸ pfu/ml, FAdV-9Δ4-CAG-EGFP-WPRE = 3.0×10⁸ pfu/ml, and FAdV-9Δ4- EF1α-EGFP = 3.7×10⁸ pfu/ml. The appearance of CPE (Figure 19) and plaque morphology (not shown) of recFAdVs were similar to FAdV-9Δ4 in that the cells rounded and detached by 5 - 7 d.p.i. All recFAdVs expressed EGFP as seen by fluorescence microscopy.

### 3.3 Promoter activity during infection

### 3.3.1 Measuring EGFP by spectrofluorometry

EGFP expression by recFAdVs was measured between 0 - 48 h.p.i. in CH-SAH cells. Based on both spectrofluorometry and fluorescence microscopy, expression of EGFP from all recombinant viruses was low until 12 h.p.i. Strong expression of EGFP was noted between 12-30 h.p.i. but it declined after 36-48 h.p.i. Maximum fluorescence readings were measured at 30 h.p.i. for all recFAdVs (Figure 20). Cells infected with FAdV-9Δ4-CAG-EGFP showed the greatest increase in EGFP expression compared to FAdV-9Δ4-CMV-EGFP at all time-points, ranging from 12 (48 h.p.i.) to 29-fold (24 h.p.i) increase in expression. FAdV-9Δ4-EF1α-EGFP infected cells also had increased expression compared to FAdV-9Δ4-CMV-EGFP at all time-points, with a slightly lower range of 2 (12 h.p.i) to 20-fold (24 h.p.i) increase in expression. Viruses that contained a WPRE element expressed EGFP at a lower level when compared to their promoter counterparts. FAdV-9Δ4-CMV-EGFP-WPRE was the "worst performing" virus, with 2.4 (48 h.p.i.) to 17-fold (36 h.p.i.) decrease in expression compared to FAdV-9Δ4-CMV-EGFP. Although both FAdV-9Δ4-CAG-EGFP-WPRE and FAdV-9Δ4-EF1α-EGFP-WPRE performed worse than their promoter counterparts, both viruses on average expressed EGFP at a higher level than FAdV-9Δ4-CMV-EGFP. FAdV-9Δ4-CAG-EGFP-WPRE showed increased expression ranging from 1.3 (48 h.p.i) to 4.3-fold (24 h.p.i.), while FAdV-9Δ4-EF1α-EGFP-WPRE expression ranged from a 2.5-fold decrease (12 h.p.i.) to 3.6-fold increase (24 h.p.i.).

### 3.3.2 Expression of EGFP measured by Western blotting

In addition to spectrofluorometry, viral EGFP expression was also examined by Western immunoblot. Whole cell lysate was collected from infected cells at 0, 6, 12, 18, 24, 30, 36, and 48 h.p.i. At each time-point, 10 µg of total protein from each lysate was separated by SDS-PAGE, blotted, and the blot was probed using the anti-EGFP antibody (Figure 21). The molecular mass of EGFP was expected to be 27 kDa (Takebe, N., Xu, L., MacKenzie, K. L., Bertino, J. R., Moore, M. A. S. 2002. Methotrexate selection of long-term culture initiating cells following transduction of CD34+ cells with a retrovirus containing a mutated human dihydrofolate reductase gene. Cancer Gene Ther. 9(3):308-320). At early time-points of 0 and 6 h.p.i., no EGFP signal was detected from any recFAdVs. By 12 h.p.i., a band corresponding to EGFP was detected in lysate collected from FAdV-9Δ4-CAG-EGFP infected cells, which was detected up until 48 h.p.i. Cell lysates collected from FAdV-9Δ4-EF1α-EGFP, FAdV-9Δ4-CAG-EGFP-WPRE, and FAdV-9Δ4-EF1α-EGFP-WPRE also produced a detectable EGFP band from 18-36 h.p.i. In contrast, no EGFP bands were seen for FAdV-9Δ4-CMV-EGFP and FAdV-9Δ4-CMV-EGFP-WPRE infected lysates at any time-points. The negative controls, FAdV-9Δ4 infected lysate and mock infected lysate, showed no signal for EGFP throughout the time-course. Transfected CH-SAH cells containing the EGFP positive plasmid under the CMV promoter, pEGFP-N1, provided a positive signal from 12 h.p.t. onwards. At each time-point blots were probed with an anti-actin antibody to show relative levels of equal loading of all samples. Actin levels appeared similar at each time-point, suggesting equal loading, however at 48 h.p.i. actin was not detected in virus infected lysates.

**Table 6. Fold change in promoter activity of transfected CH-SAH cells at various hours post-transfection in relation to the CMV promoter. Significant activity is determined by a P value <0.05.**

| Time-point (h.p.t.) | Promoter | Δfold | SD | *P* value |
|---|---|---|---|---|
| 12 | CMV | 1.0 | - | - |
| | CMV-WPRE | 1.047 | 0.245 | 0.7526 |
| | CAG | 1.483 | 1.735 | 0.5506 |
| | CAG-WPRE | 0.479 | 0.370 | 0.0717 |
| | EF1α | 0.610 | 0.184 | 0.0015 |
| | EF1α-WPRE | 0.313 | 0.117 | 0.0005 |
| | β-actin | 0.772 | 0.418 | 0.2587 |
| | β-actin-WPRE | 0.357 | 0.097 | 0.0003 |
| | L2R | 0.566 | 0.528 | 0.1654 |
| | L2R-WPRE | 0.134 | 0.095 | 0.0001 |
| 24 | CMV | 1.0 | - | - |
| | CMV-WPRE | 1.047 | 0.116 | 0.5177 |
| | CAG | 0.409 | 0.116 | 0.0001 |
| | CAG-WPRE | 0.294 | 0.039 | 0.0001 |
| | EF1α | 0.156 | 0.076 | 0.0001 |
| | EF1α-WPRE | 0.087 | 0.018 | 0.0001 |
| | β-actin | 0.074 | 0.042 | 0.0001 |
| | β-actin-WPRE | 0.042 | 0.043 | 0.0001 |
| | L2R | 0.028 | 0.016 | 0.0001 |
| | L2R-WPRE | 0.010 | 0.002 | 0.0001 |
| 36 | CMV | 1.0 | - | - |
| | CMV-WPRE | 0.894 | 0.156 | 0.3078 |
| | CAG | 0.560 | 0.331 | 0.0180 |
| | CAG-WPRE | 0.387 | 0.139 | 0.0016 |
| | EF1α | 0.196 | 0.057 | 0.0001 |
| | EF1α-WPRE | 0.124 | 0.063 | 0.0001 |
| | β-actin | 0.056 | 0.037 | 0.0001 |
| | β-actin-WPRE | 0.015 | 0.007 | 0.0001 |
| | L2R | 0.014 | 0.013 | 0.0001 |
| | L2R-WPRE | 0.002 | 0.001 | 0.0001 |
| 48 | CMV | 1.0 | - | - |
| | CMV-WPRE | 0.922 | 0.135 | 0.3758 |
| | CAG | 0.431 | 0.036 | 0.0001 |
| | CAG-WPRE | 0.303 | 0.050 | 0.0001 |
| | EF1α | 0.187 | 0.063 | 0.0001 |
| | EF1α-WPRE | 0.085 | 0.022 | 0.0001 |
| | β-actin | 0.032 | 0.017 | 0.0001 |
| | β-actin-WPRE | 0.013 | 0.005 | 0.0001 |
| | L2R | 0.015 | 0.014 | 0.0001 |
| | L2R-WPRE | 0.001 | 0.001 | 0.0001 |
| 60 | CMV | 1.0 | - | - |
| | CMV-WPRE | 0.851 | 0.123 | 0.1039 |
| | CAG | 0.572 | 0.257 | 0.1423 |
| | CAG-WPRE | 0.392 | 0.191 | 0.0053 |
| | EF1α | 0.254 | 0.054 | 0.0026 |
| | EF1α-WPRE | 0.124 | 0.021 | 0.0001 |
| | β-actin | 0.033 | 0.007 | 0.0001 |
| | β-actin-WPRE | 0.015 | 0.004 | 0.0001 |
| | L2R | 0.002 | 0.001 | 0.0001 |
| | L2R-WPRE | 0.001 | 0.001 | 0.0001 |
| 72 | CMV | 1.0 | - | - |
| | CMV-WPRE | 0.643 | 0.056 | 0.0004 |
| | CAG | 0.591 | 0.137 | 0.0521 |
| | CAG-WPRE | 0.434 | 0.164 | 0.0040 |
| | EF1α | 0.296 | 0.082 | 0.0067 |
| | EF1α-WPRE | 0.234 | 0.119 | 0.0004 |
| | β-actin | 0.030 | 0.002 | 0.0001 |
| | β-actin-WPRE | 0.028 | 0.018 | 0.0001 |
| | L2R | 0.002 | 0.001 | 0.0001 |
| | L2R-WPRE | 0.003 | 0.003 | 0.0001 |

### EXAMPLE 4

### Other embodiments of recombinant FAdV-9

Figure 22 shows other embodiments of recombinant FAdV-9 wherein ORF1 and 2 are deleted at the left end of the genome and replaced with HA of H7 coding sequences and wherein ORF19, ORF11 or TR2 are deleted at the right end of the genome and replaced with a HN cassette, HN-IRES-HA of H5 cassette or HA of H5 cassette, wherein IRES (Internal Ribosomal Entry Site) allows for translation initiation in the middle of a messenger RNA (mRNA) sequence as part of the greater process of protein synthesis. The IRES used (SEQ ID NO: 41) is from a Canadian isolate of avian encephalomyelitis virus isolate (AEV-IRES)

While the present disclosure has been described with reference to what are presently considered to be the preferred examples, it is to be understood that the disclosure is not limited to the disclosed examples. To the contrary, the disclosure is intended to cover various modifications and equivalent arrangements included within the scope of the appended claims.

### REFERENCES:

Huebner, R.J., Rower W.P., Schatten, W.E., Smith, R.R, & Thomas, L.B. (1956). Studies on the use of viruses in the treatment of carcinoma of the cervix. Cancer 9(6), 1211-1218;
Cody, J. J. & Douglas, J. T. (2009). Armed replicating adenoviruses for cancer virotherapy. Cancer Gene Ther 16, 473-488;
Yamamoto, M. & Curiel, D. T. (2010). Current issues and future directions of oncolytic adenoviruses. Mol Ther 18, 243-250;
Lasaro, M. O. & Ertl, H. C. J. (2009). New Insights on Adenovirus as Vaccine Vectors. Molecular Therapy 17, 1333-1339;
Buchbinder, S. P., Mehrotra, D. V., Duerr, A., Fitzgerald, D. W., Mogg, R., Li, D., Gilbert, P. B., Lama, J. R., Marmor, M. & other authors. (2008). Efficacy assessment of a cell-mediated immunity HIV-1 vaccine (the Step Study): a double-blind, randomised, placebo-controlled, test-of-concept trial. Lancet 372, 1881-1893;
McElrath, M. J., De Rosa, S. C., Moodie, Z., Dubey, S., Kierstead, L., Janes, H., Defawe, O. D., Carter, D. K., Hural, J. & other authors. (2008). HIV-1 vaccine-induced immunity in the test-of-concept Step Study: a case-cohort analysis. Lancet 372, 1894-1905;
Cody & Douglas, 2009; Gallo, P., Dharmapuri, S., Cipriani, B. & Monaci, P. (2005). Adenovirus as vehicle for anticancer genetic immunotherapy. Gene Ther 12, S84-S91;
Shashkova, E. V., Cherenova, L. V., Kazansky, D. B. & Doronin, K. (2005). Avian adenovirus vector CELO-TK displays anticancer activity in human cancer cells and suppresses established murine melanoma tumors. Cancer Gene Ther 12, 617-626;
Barouch, D. H. (2008). Challenges in the development of an HIV-1 vaccine. Nature 455, 613-619;
Sharma, A., Tandon, M., Ahi, Y. S., Bangari, D. S., Vemulapalli, R. & Mittal, S. K. (2009). Evaluation of Cross-Reactive Humoral and Cell-Mediated Immune Responses among Human, Bovine and Porcine Adenoviruses. Molecular Therapy 17, 113;
Adair, B. & Fitzgerald, S. (2008). Group I Adenovirus Infections. In Diseases of Poultry, 12th ed, pp. 252-266. Edited by Y. Saif, A. Fadly, J. Glisson, L. McDougald, L. Nolan & D. Swayne. Hoboken, NJ: Wiley-Blackwell;
Benkö, M., Harrach, B., Both, G., Russell, W., Adair, B., Ádam, É., de Jong, J., Hess, M., Johnson, M. & other authors. (2005). Family Adenoviridae. In Virus taxonomy Eighth report of the International Committee on the Taxonomy of Viruses, pp. 213-228. Edited by C. Fauquet, M. Mayo, J. Maniloff, U. Desselberger & L. Ball. San Diego, CA: Elsevier Academic Press; Corredor, J. C. & Nagy, E. (2010b). The non-essential left end region of the fowl adenovirus 9 genome is suitable for foreign gene insertion/replacement. Virus Res 149, 167-174;
Ojkic, D. & Nagy, E. (2003). Antibody response and virus tissue distribution in chickens inoculated with wild-type and recombinant fowl adenoviruses. Vaccine 22, 42-48;
Francois, A., Chevalier, C., Delmas, B., Eterradossi, N., Toquin, D., Rivallan, G. H. & Langlois, P. (2004). Avian adenovirus CELO recombinants expressing VP2 of infectious bursal disease virus induce protection against bursal disease in chickens. Vaccine 22, 2351-2360;
Sheppard, M., Werner, W., Tsatas, E., McCoy, R., Prowse, S. & Johnson, M. (1998). Fowl adenovirus recombinant expressing VP2 of infectious bursal disease virus induces protective immunity against bursal disease. Arch Virol 143, 915-930;
Johnson, M. A., Pooley, C., Ignjatovic, J. & Tyack, S. G. (2003). A recombinant fowl adenovirus expressing the S1 gene of infectious bronchitis virus protects against challenge with infectious bronchitis virus. Vaccine 21, 2730-2736;
Chiocca, S., Kurzbauer, R., Schaffner, G., Baker, A., Mautner, V. & Cotten, M. (1996). The complete DNA sequence and genomic organization of the avian adenovirus CELO. J Virol 70, 2939-2949;
Ojkic, D. & Nagy, E. (2000). The complete nucleotide sequence of fowl adenovirus type 8. J Gen Virol 81, 1833-1837;
Corredor, J. C., Garceac, A., Krell, P. J. & Nagy, E. (2008). Sequence comparison of the right end of fowl adenovirus genomes. Virus genes 36, 331-344;
Corredor, J. C., Krell, P. J. & Nagy, E. (2006). Sequence analysis of the left end of fowl adenovirus genomes. Virus genes 33, 95-106;
Bangari, D. S. & Mittal, S. K. (2006). Development of nonhuman adenoviruses as vaccine vectors. Vaccine 24, 849-862;
Ferreira, T. B., Alves, P. M., Aunins, J. G. & Carrondo, M. J. T. (2005). Use of adenoviral vectors as veterinary vaccines. Gene Ther 12, S73-S83;
Corredor, J. C. & Nagy, E. (2010a) A region at the left end of the fowl adenovirus 9 genome that is non-essential in vitro has consequences in vivo. J Gen Virol 91(1), 51-58;

## Claims

1. A fowl adenovirus 9 (FAdV-9) recombinant viral vector comprising a deletion of a non-essential region selected from ORF19 and ORF17.

2. The FAdV-9 vector of claim 1, wherein the vector comprises a deletion of ORF17.

3. The FAdV-9 vector of claim 1, wherein the vector comprises a deletion of ORF19.

4. The FAdV-9 vector of any one of claims 1 to 3, wherein the vector further comprises a deletion of one or more of the following non-essential regions: ORFO, ORF1 and ORF2.

5. The FAdV-9 viral vector of claim 4, wherein the vector comprises:
i) a nucleotide sequence with at least 70% sequence identity to the sequence shown in SEQ ID NO: 1, wherein nucleotides 575 to 2753 and 38,807 to 42,398 have been deleted,
ii) a nucleotide sequence with at least 70% sequence identity to the sequence shown in SEQ ID NO: 1, wherein nucleotides 847 to 2753 and 38,807 to 42,398 have been deleted,
iii) a nucleotide sequence with at least 70% sequence identity to the sequence shown in SEQ ID NO: 1, wherein nucleotides 847 to 2753 and 34,220 to 36,443 have been deleted, or
iv) a nucleotide sequence with at least 70% sequence identity to the sequence shown in SEQ ID NO: 1, wherein nucleotides 847 to 2753, 34,220 to 36,443 and 38,807 to 40,561 have been deleted or wherein nucleotides 847 to 2753, 34,220 to 36,443 and 41,461 to 42,398 have been deleted.

6. The FAdV-9 viral vector of any one of claims 1 to 5, comprising one or more exogenous nucleotide sequences coding for one or more polypeptides of interest, wherein the one or more exogenous nucleotide sequences are inserted into a non-essential region of the FAdV-9 viral vector.

7. The FAdV-9 viral vector of claim 6, wherein the vector is a dual vector comprising two exogenous nucleotide sequences coding for two polypeptides of interest.

8. The FAdV-9 viral vector of any one or claims 1 to 7, comprising an exogenous nucleotide sequence coding for at least one antigenic site of a disease of concern, wherein the exogenous nucleotide sequence is inserted into a non-essential region of the FAdV-9 viral vector.

9. The FAdV-9 viral vector of claim 8, wherein the exogenous nucleotide sequence is:
i) selected from antigenic sequences against influenza, infectious laryngotracheitis, infectious bronchitis, infectious bursal disease (Gumboro), hepatitis, viral rhinotracheitis, infectious coryza, Mycoplasma hyopneumoniae, pasteurellosis, Porcine Respiratory and Reproductive Syndrome (PRRS), circovirus, bordetellosis, parainfluenza and any other antigen which size allows its insertion into the corresponding viral vector,
ii) selected from antigenic sequences against Avian influenza, Laryngotracheitis (LT), Newcastle disease (NDV), infectious anemia, Inclusion bodies hepatitis, Infectious Bronchitis (IB), Metapneumovirus (MPV) and Gumboro, or
iii) comprises at least one sequence selected from SEQ ID NOs: 10-21 or a homolog thereof having at least 85% sequence identity thereto.

10. The viral vector of any one of claims 6 to 9, wherein the exogenous nucleotide sequence is operably linked to a control sequence, optionally a promoter sequence.

11. A host cell comprising the viral vector of any one of claims 1 to 10.

12. A method for producing the viral vector of any one of claims 6 to 10, comprising the steps of:
a) providing the FAdV-9 viral vector of any one of claims 1 to 5;
b) optionally amplifying the exogenous nucleotide sequence;
c) inserting the exogenous nucleotide sequence in the viral vector; and,
d) introducing the infectious clone thus produced into a suitable cell line.

13. The method of claim 12, wherein the exogenous nucleotide sequence:
i) is selected from antigenic sites sequences against influenza, infectious laryngotracheitis, infectious bronchitis, bursa of Fabricius' infection (Gumboro), hepatitis, viral rhinotracheitis, infectious coryza, Mycoplasma hyopneumoniae, pasteurellosis, Porcine Respiratory and Reproductive Syndrome (PRRS), circovirus, bordetellosis, parainfluenza, and any other antigen which size allows its insertion into the corresponding viral vector, optionally wherein the exogenous nucleotide sequence is selected from antigenic sites sequences against Avian influenza, Laryngotracheitis (LT), Newcastle disease (NDV), infectious anemia, Inclusion bodies, Infectious Bronchitis (IB), Metapneumovirus (MPV) and Gumboro, or
ii) comprises a sequence corresponding to at least one sequence selected from SEQ ID NOs: 10-21 or a homolog thereof.

14. An immunogenic composition comprising at least the FAdV-9 viral vector of claim 8 or 9 with an exogenous nucleotide sequence coding for at least one antigenic site of a disease of concern inserted therein, optionally further comprising a pharmaceutically acceptable carrier and/or an adjuvant.

15. An immunogenic composition of claim 14 for use in generating an immunogenic response in a subject.

## Patentansprüche

1. Rekombinanter viraler Geflügel-Adenovirus 9 (FAdV-9)-Vektor, umfassend eine Deletion einer nicht-essentiellen Region, ausgewählt aus ORF19 und ORF17.

2. FAdV-Vektor nach Anspruch 1, wobei der Vektor eine Deletion von ORF17 umfasst.

3. FAdV-Vektor nach Anspruch 1, wobei der Vektor eine Deletion von ORF19 umfasst.

4. FAdV-Vektor nach einem jeglichen der Ansprüche 1 bis 3, wobei der Vektor ferner eine Deletion einer oder mehrerer der nachstehenden nicht-essentiellen Regionen umfasst: ORF0, ORF1 und ORF2.

5. Viraler FAdV-Vektor nach Anspruch 4, wobei der Vektor umfasst:
i) eine Nukleotidsequenz mit einer Sequenzidentität von mindestens 70% zu der in SEQ ID NO: 1 gezeigten Sequenz, wobei Nukleotide 575 bis 2753 und 38 807 bis 42 398 deletiert wurden,
ii) eine Nukleotidsequenz mit einer Sequenzidentität von mindestens 70% zu der in SEQ ID NO: 1 gezeigten Sequenz, wobei Nukleotide 847 bis 2753 und 38 807 bis 42 398 deletiert wurden,
iii) eine Nukleotidsequenz mit einer Sequenzidentität von mindestens 70% zu der in SEQ ID NO: 1 gezeigten Sequenz, wobei Nukleotide 847 bis 2753 und 34 220 bis 36 443 deletiert wurden, oder
iv) eine Nukleotidsequenz mit einer Sequenzidentität von mindestens 70% zu der in SEQ ID NO: 1 gezeigten Sequenz, wobei Nukleotide 847 bis 2753, 34 220 bis 36 443 und 38 807 bis 40 561 deletiert wurden oder wobei Nukleotide 847 bis 2753, 34 220 bis 36 443 und 41 461 bis 42 398 deletiert wurden.

6. Viraler FAdV-Vektor nach einem jeglichen der Ansprüche 1 bis 5, der eine oder mehrere exogene Nukleotidsequenzen, die für ein oder mehrere Polypeptide von Interesse kodieren, umfasst, wobei die eine oder mehreren exogenen Nukleotidsequenzen in eine nicht-essentielle Region des viralen FAdV-Vektors eingefügt sind.

7. Viraler FAdV-Vektor nach Anspruch 6, wobei der Vektor ein dualer Vektor ist, der zwei exogene Nukleotidsequenzen, die für zwei Polypeptide von Interesse kodieren, umfasst.

8. Viraler FAdV-Vektor nach einem jeglichen der Ansprüche 1 bis 7, der eine exogene Nukleotidsequenz, die für mindestens eine antigene Stelle einer besorgniserregenden Erkrankung kodiert, umfasst, wobei die exogene Nukleotidsequenz in eine nicht-essentielle Region des viralen FAdV-Vektors eingefügt ist.

9. Viraler FAdV-Vektor nach Anspruch 8, wobei die exogene Nukleotidsequenz:
i) aus antigenen Sequenzen gegen Influenza, infektiöse Laryngotracheitis, infektiöse Bronchitis, infektiöse Bursitis (Gumboro), Hepatitis, virale Rhinotracheitis, infektiöse Coryza, Mycoplasma hyopneumoniae, Pasteurellose, porzines respiratorisches und reproduktives Syndrom (PRRS), Circovirus, Bordetellose, Parainfluenza und ein jegliches andere Antigen, dessen Größe seine Einfügung in den entsprechenden viralen Vektor erlaubt, ausgewählt ist,
ii) aus antigenen Sequenzen gegen Vogelgrippe, Laryngotracheitis (LT), Newcastle-Krankheit (NDV), infektiöse Anämie, Einschlusskörperchen-Hepatitis, infektiöse Bronchitis (IB), Metapneumovirus (MPV) und Gumboro ausgewählt ist, oder
iii) mindestens eine Sequenz, ausgewählt aus SEQ ID NOs: 10-21 oder einem Homolog davon mit einer Sequenzidentität von mindestens 85% dazu, umfasst.

10. Viraler Vektor nach einem jeglichen der Ansprüche 6 bis 9, wobei die exogene Nukleotidsequenz operabel mit einer Kontrollsequenz, optional einer Promotorsequenz, verbunden ist.

11. Wirtszelle, die den viralen Vektor nach einem jeglichen der Ansprüche 1 bis 10 umfasst.

12. Verfahren zum Herstellen des viralen Vektors nach einem jeglichen der Ansprüche 6 bis 10, das die Schritte umfasst:
a) Bereitstellen des viralen FAdV-Vektors nach einem jeglichen der Ansprüche 1 bis 5;
b) optional Amplifizieren der exogenen Nukleotidsequenz;
c) Einfügen der exogenen Nukleotidsequenz in den viralen Vektor; und
d) Einbringen des so hergestellten infektiösen Klons in eine geeignete Zelllinie.

13. Verfahren nach Anspruch 12, wobei die exogene Nukleotidsequenz:
i) aus Sequenzen von antigenen Stellen gegen Influenza, infektiöse Laryngotracheitis, infektiöse Bronchitis, Infektion der Bursa Fabricii (Gumboro), Hepatitis, virale Rhinotracheitis, infektiöse Coryza, Mycoplasma hyopneumoniae, Pasteurellose, porzines respiratorisches und reproduktives Syndrom (PRRS), Circovirus, Bordetellose, Parainfluenza und ein jegliches andere Antigen, dessen Größe seine Einfügung in den entsprechenden viralen Vektor erlaubt, ausgewählt ist, wobei optional die exogene Nukleotidsequenz aus Sequenzen von antigenen Stellen gegen Vogelgrippe, Laryngotracheitis (LT), Newcastle-Krankheit (NDV), infektiöse Anämie, Einschlusskörperchen, infektiöse Bronchitis (IB), Metapneumovirus (MPV) und Gumboro ausgewählt ist, oder
ii) eine Sequenz umfasst, die mindestens einer Sequenz, ausgewählt aus SEQ ID NOs: 10-21 oder einem Homolog davon, entspricht.

14. Immunogene Zusammensetzung, die mindestens den viralen FAdV-9-Vektor nach Anspruch 8 oder 9, wobei darin eine exogenen Nukleotidsequenz, die für mindestens eine antigene Stelle einer besorgniserregenden Erkrankung kodiert, eingefügt ist, umfasst und optional ferner einen pharmazeutisch verträglichen Träger und/oder ein Adjuvans umfasst.

15. Immunogene Zusammensetzung nach Anspruch 14 zur Verwendung beim Erzeugen einer Immunantwort in einem Lebewesen.

## Revendications

1. Vecteur viral recombinant d'adénovirus aviaire 9 (FAdV-9) comprenant une délétion d'une région non essentielle choisie parmi ORF19 et ORF17.

2. Vecteur de FAdV-9 selon la revendication 1, ledit vecteur comprenant une délétion d'ORF17.

3. Vecteur de FAdV-9 selon la revendication 1, ledit vecteur comprenant une délétion d'ORF19.

4. Vecteur de FAdV-9 selon l'une quelconque des revendications 1 à 3, ledit vecteur comprenant en outre une délétion d'une ou plusieurs des régions non essentielles suivantes : ORF0, ORF1 et ORF2.

5. Vecteur viral de FAdV-9 selon la revendication 4, ledit vecteur comprenant :
i) une séquence nucléotidique présentant une identité de séquence d'au moins 70 % avec la séquence représentée dans SEQ ID N° : 1, les nucléotides 575 à 2753 et 38 807 à 42 398 ayant été supprimés,
ii) une séquence nucléotidique présentant une identité de séquence d'au moins 70 % avec la séquence représentée dans SEQ ID N° : 1, les nucléotides 847 à 2753 et 38 807 à 42 398 ayant été supprimés,
iii) une séquence nucléotidique présentant une identité de séquence d'au moins 70 % avec la séquence représentée dans SEQ ID N° : 1, les nucléotides 847 à 2753 et 34 220 à 36 443 ayant été supprimés, ou
iv) une séquence nucléotidique présentant une identité de séquence d'au moins 70 % avec la séquence représentée dans SEQ ID N° : 1, les nucléotides 847 à 2753 et 34 220 à 36 443 et 38 807 à 40 561 ayant été supprimés ou les nucléotides 847 à 2753 et 34 220 à 36 443 et 41 461 à 42 398 ayant été supprimés.

6. Vecteur viral de FAdV-9 selon l'une quelconque des revendications 1 à 5, comprenant une ou plusieurs séquences nucléotidiques exogènes codant un ou plusieurs polypeptides d'intérêt, lesdites une ou plusieurs séquences nucléotidiques exogènes étant insérées dans une région non essentielle du vecteur viral de FAdV-9.

7. Vecteur viral de FAdV-9 selon la revendication 6, ledit vecteur comprenant comprenant un vecteur double comprenant deux séquences nucléotidiques exogènes codant deux polypeptides d'intérêt.

8. Vecteur viral de FAdV-9 selon l'une quelconque des revendications 1 à 7, comprenant une séquence nucléotidique exogène codant au moins un site antigénique d'une maladie préoccupante, ladite séquence nucléotidique exogène étant insérée dans une région non essentielle du vecteur viral de FAdV-9.

9. Vecteur viral de FAdV-9 selon la revendication 8, ladite séquence nucléotidique exogène :
i) étant choisie parmi les séquences antigéniques contre la grippe, la laryngotrachéite infectieuse, la bronchite infectieuse, la bursite infectieuse (Gumboro), l'hépatite, la rhinotrachéite virale, le coryza infectieux, le Mycoplasma hyopneumoniae, la pasteurellose, le syndrome dysgénésique et respiratoire du porc (SDRP), le circovirus, la boretellose, la parainfluenza et tout autre antigène dont la taille permet son insertion dans le vecteur viral correspondant,
ii) étant choisie parmi les séquences antigéniques contre la grippe aviaire, la laryngotrachéite (LT), la maladie de Newcastle (NDV), l'anémie infectieuse, l'hépatite à corps d'inclusion, la bronchite infectieuse (BI), le métapneumovirus (MPV) et le Gumboro, ou
iii) comprenant au moins une séquence choisie parmi les SEQ ID N° : 10 à 21 ou un homologue de celle-ci présentant une identité de séquence d'au moins 85 % avec celle-ci.

10. Vecteur viral selon l'une quelconque des revendications 6 à 9, ladite séquence nucléotidique exogène étant fonctionnellement liée à une séquence de contrôle, éventuellement une séquence promotrice.

11. Cellule hôte comprenant le vecteur viral selon l'une quelconque des revendications 1 à 10.

12. Méthode de production du vecteur viral selon l'une quelconque des revendications 6 à 10, comprenant les étapes de :
a) fourniture du vecteur viral de FAdV-9 selon l'une quelconque des revendications 1 à 5 ;
b) amplification éventuelle de la séquence nucléotidique exogène ;
c) insertion de la séquence nucléotidique exogène dans le vecteur viral ; et,
d) introduction du clone infectieux ainsi produit dans une lignée cellulaire appropriée.

13. Méthode selon la revendication 12, ladite séquence nucléotidique exogène :
i) étant choisie parmi les séquences de sites antigéniques contre la grippe, la laryngotrachéite infectieuse, la bronchite infectieuse, l'infection de la bourse de Fabricius (Gumboro), l'hépatite, la rhinotrachéite virale, le coryza infectieux, le Mycoplasma hyopneumoniae, la pasteurellose, le syndrome dysgénésique et respiratoire du porc (SDRP), le circovirus, la boretellose, la parainfluenza et tout autre antigène dont la taille permet son insertion dans le vecteur viral correspondant, éventuellement ladite séquence nucléotidique exogène étant choisie parmi les séquences de sites antigéniques contre la grippe aviaire, la laryngotrachéite (LT), la maladie de Newcastle (NDV), l'anémie infectieuse, les corps d'inclusion, la bronchite infectieuse (BI), le métapneumovirus (MPV) et le Gumboro, ou
ii) comprenant une séquence correspondant à au moins une séquence choisie parmi les SEQ ID N° : 10 à 21 ou un homologue de celle-ci.

14. Composition immunogène comprenant au moins le vecteur viral de FAdV-9 selon la revendication 8 ou 9 avec une séquence nucléotidique exogène codant au moins un site antigénique d'une maladie préoccupante insérée en lui, éventuellement comprenant en outre un véhicule pharmaceutiquement acceptable et/ou un adjuvant.

15. Composition immunogène selon la revendication 14 destinée à être utilisée dans la génération d'une réponse immunogène chez un sujet.
